# EUROPEAN PATENT SPECIFICATION

(11) **EP 1 058 728 B1**
(45) Date of publication and mention of the grant of the patent: **30.04.2008**
(21) Application number: 99908689.5
(22) Date of filing: 05.03.1999
(51) Int. Cl.: C12N 15/12, C12N 15/13, C07K 14/705, C07K 14/73, C07K 14/665, C07K 14/745

(54) **V-LIKE DOMAIN BINDING MOLECULES**
V-ÄHNLICHE DOMÄN-BINDENDE MOLEKÜLE
MOLECULES DE FIXATION A DOMAINE DE TYPE V

(30) Priority: 06.03.1998 AU PP221098
(43) Date of publication of application: 13.12.2000
(73) Proprietor: Diatech Pty. Ltd., Brisbane, QLD 4001 (AU)
(72) Inventor: COIA, Gregory, Brunswick, VIC 3056 (AU); GALANIS, Maria, Glen Waverley, VIC 3150 (AU); HUDSON, Peter, John, Blackburn, VIC 3130 (AU); IRVING, Robert, Alexander, Mulgrave, VIC 3170 (AU); NUTTALL, Stewart, Douglas, Ivanhoe, VIC 3079 (AU)
(74) Representative: Bannerman, David Gardner
(86) International application number: PCT/AU1999/000136
(87) International publication number: WO 1999/045110

(56) References cited:
- WO-A-91/10438
- WO-A-92/01787
- WO-A-98/33513
- LINSLEY P S ET AL: "Binding stoichiometry of the cytotoxic T lymphocyte-associated Molecule-4 (CTLA-4)" JOURNAL OF BIOLOGICAL CHEMISTRY, AMERICAN SOCIETY OF BIOLOGICAL CHEMISTS, BALTIMORE, MD, US, vol. 270, no. 25, 23 June 1995 (1995-06-23), pages 15417-15424, XP002129410 ISSN: 0021-9258
- JUNG S. and PLUCKTHUN A., PROTEIN ENG., 1997, 10(8), pages 959-966, XP000971779.
- PATTEN et al., J. IMMUNOL., 1993, 150(6), pages 2281-2294, XP000978260.
- PEACH R.J. et al., J. EXP. MED., 1994, 180(6), pages 2049-2058, XP000199779.
- DAVIES J. and REICHMAN L., PROTEIN ENG., 1996, 9(6), pages 531-537, XP000971767, MEDLINE ABSTRACT.

## Description

### Field of the Invention

The present invention relates to V-like Domain binding molecules with affinities for target molecules. The present invention also relates to compositions comprising these V-like domain binding molecules.
The present invention also relates to a method for selecting V-like Domain binding molecules with novel binding affinities and/or specificities.

### Background of the Invention

### Immunoglobulin Superfamily - Antibody Variable (V) Domains

Antibodies are the paradigm of specific high-affinity binding reagents and provide an antigen binding site by interaction of variable heavy (V_{H}) and variable light (V_{L}) immunoglobulin domains. The binding interface is formed by six surface polypeptide loops, termed complementarity determining regions (CDRs), three from each variable domain, which are highly variable and combined provide a sufficiently large surface area for interaction with antigen. Specific binding reagents can be formed by association of only the V_{H} and V_{L} domains into an Fv module. Bacterial expression is enhanced by joining the V-domains with a linker polypeptide into a single-chain scFv molecule. "Humanisation" of recombinant antibodies by grafting murine CDR loop structures onto a human Fv framework is disclosed by Winter et al EP-239400.

Methods to improve the expression and folding characteristics of single-chain Fv molecules were described by Nieba et al (1997). The properties of single V-domains, derived from natural mammalian antibodies, have been described by Gussow et al in WO/90/05144 and EP 0368G84B1 and by Davis et al in WO/91/08482. Single camelid V-domains have been described by Harners et al in WO/96/34103 and in WO/94/25591. A method for reducing the hydrophobicity of the surface of a human V_{H} domain by replacing human amino acid sequences with camelid amino acid sequences was described by Davies and Riechmann (1994). Methods to exchange other regions of human V_{H} sequences with camel sequences to further enhance protein stability, including the insertion of cysteine residues in CDR loops, were described by Davies and Riechmann (1996).

Several attempts to engineer high-affinity single domain binding reagents using either the V_{H} or V_{L} domains alone have been unsuccessful, due to lack of binding specificity and the inherent insolubility of single domains in the absence of the hydrophobic face where the V_{H} and V_{L} domains interact (Kortt et al, 1995).

### T-cell Receptor Variable (V) Domains

The T-cell receptor has two V-domains that combine into a structure similar to the Fv module of an antibody that results from combination of the VH and VL domains. Novotny et al (1991) described how the two V-domains of the T-cell receptor (termed alpha and beta) can be fused and expressed as a single chain polypeptide and, further, how to alter surface residues to reduce the hydrophobicity directly analogous to an antibody scFv. Other publications describe the expression characteristics of single-chain T-cell receptors comprising two V-alpha and V-beta domains (Wulfing and Pluckthun,1994; Ward, 1991).

### Non-antibody ligands - CTLA-4 and CD28 V-like Domains

There are a class of non-antibody ligands which bind to specific binding partners which also comprise V-like domains. These V-like domains are distinguished from those of antibodies or T-cell receptors because they have no propensity to join together into Fv-type molecules. These non-antibody ligands provide an alternative framework for the development of novel binding moieties with high affinities for target molecules. Single domain V-like binding molecules derived from these non-antibody ligands which are soluble are therefore desirable. Examples of suitable non-antibody ligands are CTLA-4, CD28 and ICOS (Hutloff et al, 1999).

Cytotoxic T-lymphocyte associated antigen 4 (CTLA-4) and the homologous cell-surface proteins CD28 and ICOS, are involved in T-cell regulation during the immune response. CTLA-4 is a 44 kDa homodimer expressed primarily and transiently on the surface of activated T-cells, where it interacts with CD80 and CD86 surface antigens on antigen presenting cells to effect regulation of the immune response (Waterhouse et al. 1996, van der Merwe et al. 1997). CD28 is a 44kDa homodimer expressed predominantly on T-cells and, like CTLA-4, interacts with CD80 and CD86 surface antigens on antigen presenting cells to effect regulation of the immune response (Linsley et al.1990). Current theory suggests that competition between CTLA-4 and CD28 for available ligands controls the level of immune response, for example, gene deletion of CTLA-4 in knock-out mice results in a massive over-proliferation of activated T-cells (Waterhouse et al. 1995).

Each CTLA-4 monomeric subunit consists of an N-terminal extracellular domain, transmembrane region and C-terminal intracellular domain. The extracellular domain comprises an N-terminal V-like domain (VLD; of approximately 14 kDa predicted molecular weight by homology to the immunoglobulin superfamily) and a stalk of about 10 residues connecting the VLD to the transmembrane region. The VLD comprises surface loops corresponding to CDR-1, CDR-2 and CDR-3 of an antibody V-domain (Metzler 1997). Recent structural and mutational studies on CTLA-4 suggest that binding to CD80 and CD86 occurs via the VLD surface formed from A'GFCC' V-like beta-strands and also from the highly conserved MYPPPY sequence in the CDR3-like surface loop (Peach et al. 1994; Morton et al. 1996; Metzler et al. 1997). Dimerisation between CTLA-4 monomers occurs through a disulphide bond between cysteine residues (Cys¹²⁰) in the two stalks, which results in tethering of the two extracellular domains, but without any apparent direct association between V-like domains (Metzler et al. 1997). Dimerisation appears to contribute exclusively to increased avidity for the ligands.

### In vitro Expression of Soluble Forms of CTLA-4.

Neither the extracellular domains nor V-like domains (VLDs) of human CTLA-4 molecule have been successfully expressed as soluble monomers in bacterial cells, presumably due to aggregation of the expressed proteins (Linsley et al, 1995). Expression of the extracellular N-terminal domain (Met¹ to Asp¹²⁴, comprising Cys¹²⁰) in *E.coli* results in production of a dimeric 28 kDa MW protein, in which two CTLA-4 V-like domains are joined by a disulphide linkage at Cys¹²⁰. Truncation at Val¹¹⁴ removes these cysteines and was intended to enable expression of a 14 kDa VLD in soluble, monomeric form. However, the product aggregated and it was concluded that hydrophobic sites, which were normally masked by glycosylation, were now exposed and caused aggregation (Linsley et al, 1995).

There have been some reports of successful expression of monomeric, glycosylated CTLA-4 extracellular domains in eukaryotic expression systems (ie CHO cells and the yeast Pichia pastoris; Linsley et al. 1995; Metzler et al. 1997; Gerstmayer et al. 1997). Glycosylation in these eukaryotic expression systems is presumed to occur at the two N-linked glycosylation sites in the VLD (Asn76 and Asn108). However, high yields have only been described for expression of a gene encoding a CTLA-4 VLD fused to Ig-CH2/CH3 domains which produces a dimeric recombinant protein with 2 CTLA-4 VLDs attached to an Fc subunit (WO 95/01994 and AU 16458/95). AU 60590/96 describes mutated forms of CTLA-4 VLDs with single amino acid replacements of the first tyrosine (Y) in the MYPPPY surface loop which retain and modifies the affinity for the natural CD80 and CD86 ligands. AU 60590/96 describes the preferred soluble form of CTLA-4 VLDs as a recombinant CTLA-4/Ig fusion protein expressed in eukaryotic cells and does not solve the aggregation problem in prokaryote expression systems. EP 0757099A2 describes the use of CTLA-4 mutant molecules, for example the effect of changes on ligand binding of mutations in the CDR3-like loop.

### Summary of the Invention

The present inventors have now developed novel binding molecules derived from the V-like domains (VLDs) of non-antibody ligands such as CTLA-4, CD28 and ICOS. Replacement of CDR loop structures within the VLDs results unexpectedly in the production of monomeric, correctly folded molecules with altered binding specificities and improved solubility.

Accordingly, in a first aspect the present invention provides a binding moiety comprising at least one monomeric non-antibody V-like domain (VLD), the at least one monomeric V-like domain being characterised in that at least one CDR loop structure or part thereof is modified or replaced such that
(i) the size of the CDR loop structure is increased when compared with corresponding CDR loop structure in the unmodified VLD; and/or
(ii) the modification or replacement results in the formation of a disulphide bond within or between one or more of the CDR loop structures.

Within the context of the present invention, the modification or replacement may involve any change to one or more physical characteristics (such as size, shape, charge, hydrophobicity etc) of the at least one CDR loop structure. The modification or replacement may result in a reduction in the size of the at least one CDR loop structure. The modification or replacement improves the solubility of the modified VLD when compared with the unmodified VLD.

In a second aspect, the present invention provides a binding moiety comprising at least one monomeric V-like domain (VLD) derived from a non-antibody ligand, the at least one monomeric V-like domain being characterised in that at least one CDR loop structure or part thereof is modified or replaced such that
(i) the size of the CDR loop structure is altered when compared with corresponding CDR loop structure in the unmodified VLD: and/or
(ii) the modification or replacement results in the formation of a disulphide bond within or between one or more of the CDR loop structures.

In a preferred embodiment of the second aspect, the size of the CDR loop structure is increased by at least two, more preferably at least three, more preferably at least six and more preferably at least nine amino acid residues.

In a further preferred embodiment, the modified binding moiety of the first or second aspect of the present invention also exhibits an altered binding affinity or specificity when compared with the unmodified binding moiety. Preferably, the effect of replacing or modifying the CDR loop structure is to reduce or abolish the affinity of the VLD to one or more natural ligands of the unmodified VLD. Preferably, the effect of replacing or modifying the CDR loop structure is also to change the binding specificity of the VLD. Thus it is preferred that the modified VLD binds to a specific binding partner which is different to that of the unmodified VLD.

The phrase "V-like domain" or "VLD" is intended to refer to a domain which has similar structural features to the variable heavy (V_{H}) or variable light (V_{L}) antibody. These similar structural features include CDR loop structures. By "CDR loop structures" we mean surface polypeptide loop structures or regions like the complementarity determining regions in antibody V-domains.

The phrase "non-antibody ligand" is intended to refer to any ligand which binds to a specific binding partner and which is not an antibody or a T-cell receptor. Examples of suitable non-antibody ligands are T-cell surface proteins such as CTLA-4, CD28 and ICOS. It will be appreciated by those skilled in the art that other non-antibody ligands which may provide V-like domains suitable for the invention are other T-cell surface proteins such as CD2. CD4, CD7 and CD16; B cell surface proteins such as CD19, CD79a, CD22, CD33, CD80 and CD86; adhesion molecules such as CD48, CD541CAM and CD58. These molecules, which are listed in Table 1, provide a non-exhaustive list of structures which may form the basis for the single domain binding molecules of the present invention.

The phrase "V-like domain derived from a non-antibody ligand" is intended to encompass chimeric V-like domains which comprise at least part of a V-like domain derived from a non-antibody ligand.

**TABLE 1: NON-ANTIBODY LIGANDS**

| | | |
|---|---|---|
| Molecule | Size | Structure |

| Tcell Surface Proteins | | |
|---|---|---|
| CD2 | 45-58kDa | VC¹ domains |
| CD4 | 55kDa | V2C2 |
| CD7 | 40kDa | V domain |
| CD16 | 50-65kDa | 2x C domains |
| | | |

| B cell Surface Proteins | | |
|---|---|---|
| CD19 | 95kDa | 2x C domains |
| CD79a | 33kDa | |
| CD22 | 130-140kDa | 1xV 6xC domains |
| CD33 | 67kDa | VC domain |
| CD80 | 60kDa | VC domain |
| CD86 | 60kDa | VC domain |
| | | |

| Adhesion molecules | | |
|---|---|---|
| CD48 | 45kDa | VC domain |
| CD541CAM | 85-110kDa | |
| CD58 | 55-70kDa | VC domain |

| | | |
|---|---|---|
| 1 V = variable 1g domain, C = constant domain | | |

These molecules are discussed in (1) The Leucocyte Antigen Facts Book. 1993. Eds Barclay et al., Academic Press. London: and (2) CD Antigens 1996 (1997) Immunology Today 18. 100-101.

The "solubility" of modified binding moieties of the present invention correlates with the production of correctly folded, monomeric domains. The solubility of the modified VLDs may therefore be assesed by HPLC. For example, soluble (monomeric) VLDs will give rise to a single peak on the HPLC chromatograph, whereas insoluble (eg. multimeric and aggregated) VLDs will give rise to a plurality of peaks. A person skilled in the art will therefore be able to detect an increase in solubility of modified VLDs using routine HPLC techniques.

It will be appreciated that the binding moieties of the present invention may be coupled together, either chemically or genetically, to form multivalent or multifunctional reagents. For example, the addition of C-terminal tails, such as in the native CTLA-4 with Cys¹²⁰, will result in a dimer.

The binding moieties of the present invention may also be coupled to other molecules for various diagnostic formulations. For example, the VLDs may comprise a C-terminal polypeptide tail or may be coupled to streptavidin or biotin for multi-site *in vitro* assays. The VLDs may also be coupled to radioisotopes, dye markers or other imaging reagents for *in vivo* detection and/or localisation of cancers, blood clots, etc. The VLDs may also be immobilised by coupling onto insoluble devices and platforms for diagnostic and biosensor applications.

In a most preferred embodiment of the first aspect of the present invention, the V-like domain is derived from the extracellular domain of the CTLA-4 molecule or the CD28 molecule. In a further preferred embodiment one or more surface loops of the CTLA-4 V-like domain and preferably the CDR-1, CDR-2 or CDR-3 loop structures are replaced with a polypeptide which has a binding affinity for a target molecule of interest. Target molecules of interest comprise, but are not limited to, drugs, steroids, pesticides, antigens, growth factors, tumour markers, cell surface proteins or viral coat proteins. It will be appreciated that these VLDs may be polyspecific, having affinities directed by both their natural surfaces and modified polypeptide loops.

In a further preferred embodiment the effect of replacing or modifying the CTLA-4, CD28 and ICOS V-like domain surface loops is to abolish the natural affinity to CD80 and CD86.

In one preferred embodiment, one or more of the CDR loop structures of the VLD are replaced with one or more CDR loop structures derived from an antibody. The antibody may be derived from any species. In a preferred embodiment, the antibody is derived from a human, rat, mouse, camel, llama or shark. The antibody or antibodies may be selected from the camel antibody cAB-Lys3 and the human anti-melanoma antibody V86.

In a further preferred embodiment, one or more of the CDR loop structures are replaced with a binding determinant derived from a non-antibody polypeptide. For example, one or more of the CDR loop structures may be replaced with a polypeptide hormone, such as somatostatin which is a 14 residue intra-disulphide bonded polypeptide important in cancer cell recognition, or with a viral protein such as the human influenza virus haemagglutinin protein.

In a further preferred embodiment the V-like domain of the binding moiety comprises CDR loop structures homologous in character to CDR loop structures found in camelid or llama antibodies. For example, the CDR loop structures may contain one or more non-conventional substitutions (eg. hydrophobic to polar in nature). In another preferred embodiment, the CDR-1 and CDR-3 loop structures may adopt non-canonical conformations which are extremely heterologous in length. The V-like domain may also possess a disulphide linkage interconnecting the CDR-1 and CDR-3 loop structures (as found in some camel V_{H}H antibodies) or the CDR-2 and CDR-3 loop structures (as found in some llama V_{H}H antibodies).

In a third aspect the present invention provides a polynucleotide encoding a binding moiety of the first or second aspect of the present invention. The polynucleotide may be incorporated into a plasmid or expression vector.

In a fourth aspect the present invention provides a prokaryotic or eukaryotic host cell transformed with a polynucleotide according to the third aspect of the present invention.

In a fifth aspect the present invention provides a method of producing a binding moiety which comprises culturing a host cell according to the fourth aspect of the present invention under conditions enabling expression of the binding moiety and optionally recovering the binding moiety.

In a preferred embodiment of the present invention the binding moiety is produced by expression in a bacterial host. Preferably, the binding moiety is unglycosylated.

In a sixth aspect the present invention provides a pharmaceutical composition comprising a binding moiety of the first or second aspect of the present invention and a pharmaceutically acceptable carrier or diluent.

In a seventh aspect the present invention provides a binding moiety or reagent according to the first or second aspect of the present invention for use in treating a pathological condition in a subject according to the first or second aspect of the present invention.

For *in vivo* applications it is preferable that VLDs are homologous to the subject of treatment or diagnosis and that any possible xenoantigens are removed. Accordingly it is preferred that VLD molecules for use in clinical applications are substantially homologous to naturally occurring human immunoglobulin superfamily members.

In an eighth aspect the present invention provides a method of selecting a binding moiety with an affinity for a target molecule which comprises screening a library of polynucleotides for expression of a binding moiety with an affinity for the target molecule, the polynucleotides encoding one or more non-antibody ligand VLDs, wherein the polynucleotides have been subjected to mutagenesis which results in a modification or replacement in at least one CDR loop structure in at least one VLD such that
(i) the size of the loop structure is increased when compared with the corresponding CDR loop structure in the unmodified VLD; and/or
(ii) the modification or replacement results in the formation of a disulphide bond within or between one or more of the CDR loop structures.

The solubility of the isolated modified VLD is improved when compared with the isolated unmodified VLD.

It will be appreciated by those skilled in the art that within the context of the eighth aspect of the present invention, any method of random or targetted mutagenesis may be used to introduce modification into the V-like domains. In a preferred embodiment, the mutagenesis is targetted mutagenesis. Preferably, the targetted mutagenesis involves replacement of at least one sequence within at least one CDR loop structure using splice overlap PCT technology.

It will also be appreciated by those skilled in the art that the polynucleotide library may contain sequences which encode VLDs comprising CDR loop structures which are substantially identical to CDR loop structures found in naturally occurring sequences which encode VLDs comprising non-naturally occurring CDR loop structures.

In a preferred embodiment of the eighth aspect of the present invention, the screening process involves displaying the modified V-like domains as gene III protein fusions on the surface of bacteriophage particles. The library may comprise bacteriophage vectors such as pHFA, fd-tet-dog or pFAB.5c containing the polynucleotides encoding the V-like domains.

In a further preferred embodiment of the eighth aspect, the screening process involves displaying the modified V-like domains in a ribosomal display selection system.

Throughout this specification, the word "comprise", or variations such as "comprises" or "comprising", will be understood to imply the inclusion of a stated element, integer or step, or group of elements, integers or steps, but not the exclusion of any other element, integer or step, or group of elements, integers or steps.

### Brief Description of the Drawings

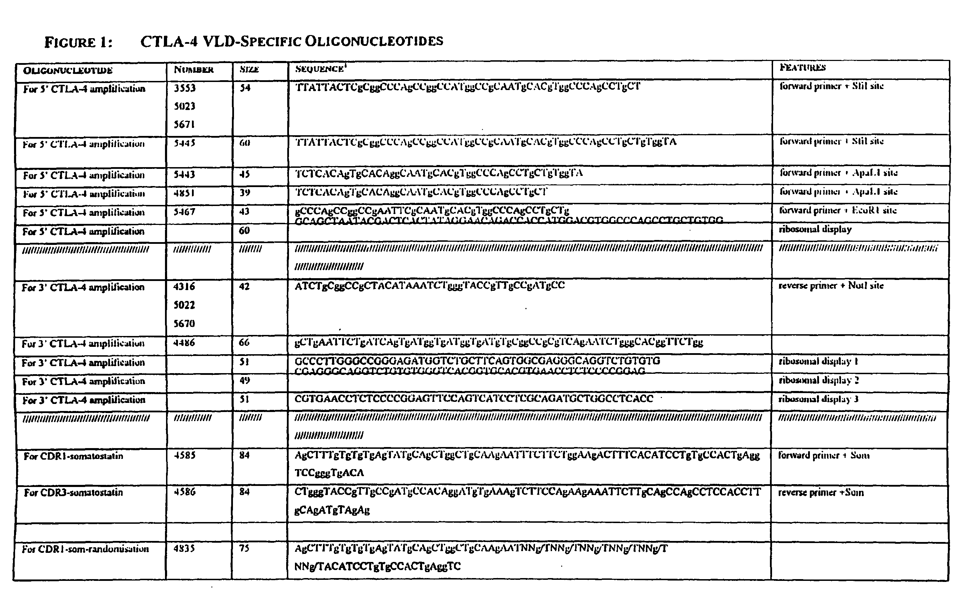

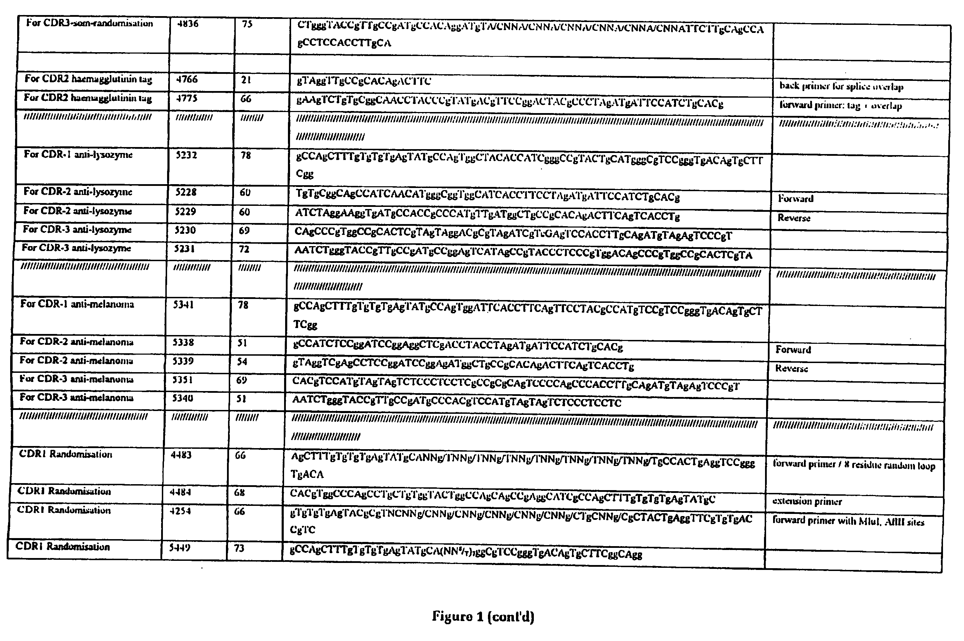

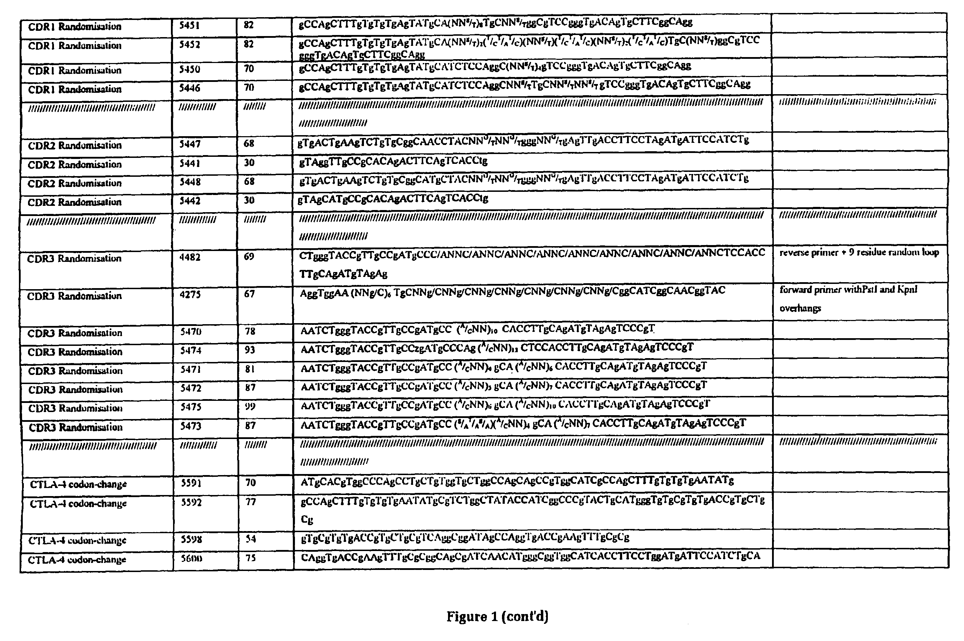

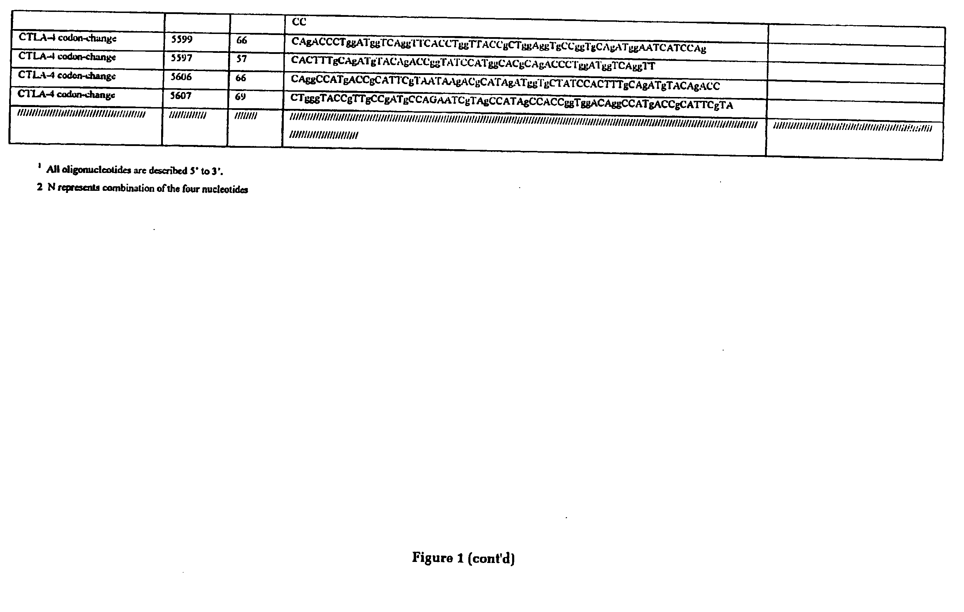

Figure 1: CTLA-4 VLD-Specific Oligonucletides.

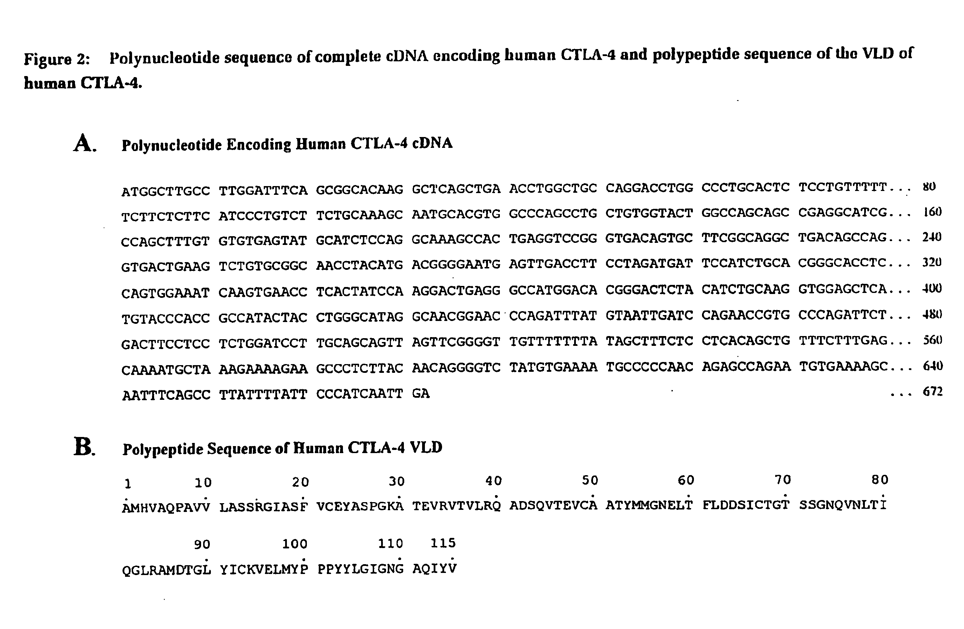

Figure 2: Polynucleotide sequence of complete cDNA encoding human CTLA-4 and polypeptide sequence of the VLD of human CTLA-4.

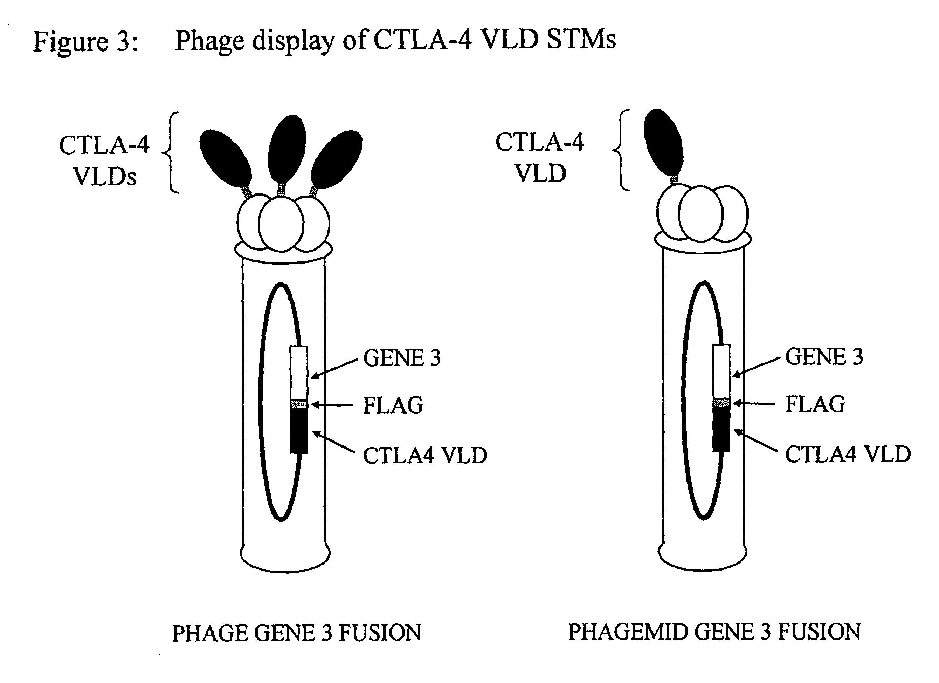

Figure 3: Display of CTLA-4 VLD STMs as gene 3 fusions on the surface of phage or phagemid. CTLA-4 VLD STMs are depicted as black spheroids; gene 3 protein is depicted as white spheroids; FLAG polypeptide is depicted in grey; genes are marked in a similar colour code and are depicted in an oval phage(mid) vector.

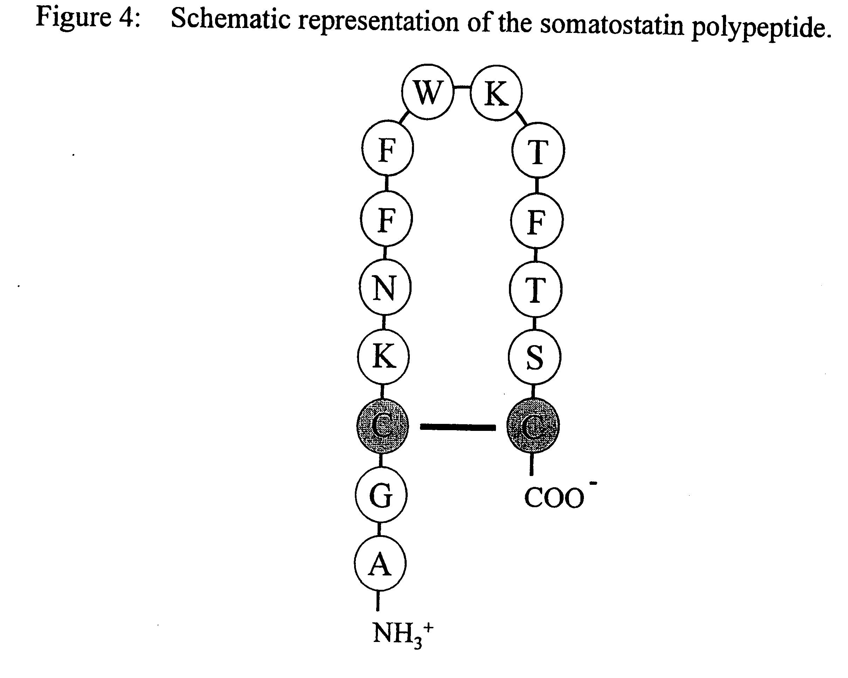

Figure 4: Schematic representation of the somatostatin polypeptide. Somatostatin (somatotropin release-inhibiting factor SRIF) is a cyclic 14-amino acid polypeptide. The cyclic nature is provided by a disulphide linkage between the cysteine residues at positions 3 and 14. The four residues which constitute the tip of the loop (Phe-Trp-Lys-Thr) are implicated in binding to members of the somatostatin receptor family.

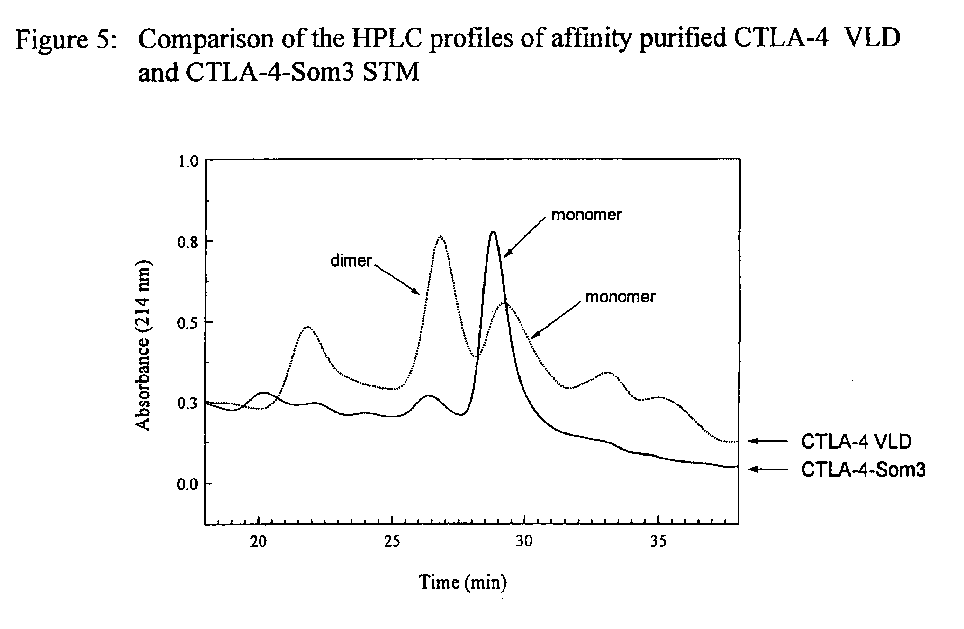

Figure 5: Size exclusion HPLC profiles of affinity purified CTLA-4 VLD and CTLA-4-Som3 STM. Recombinant human CTLA-4 proteins were expressed in E. coli host TG1 from vector pGC, purified from periplasmic extracts by anti-FLAG affinity chromatography and subjected to size exclusion chromatography on a calibrated Superose 12 HR column. The elution profiles of purified CTLA-4 VLD and CTLA-4-Som3 STM are overlayed in this graph. CTLA-4 VLD comprises tetramer (21.86 min), dimer (26.83) and monomer (29.35 min). CTLA-4-Som3 STM comprises dimer (26.34) and monomer (29.28). Traces represent absorbance at 214 nm and are given in arbitrary units.

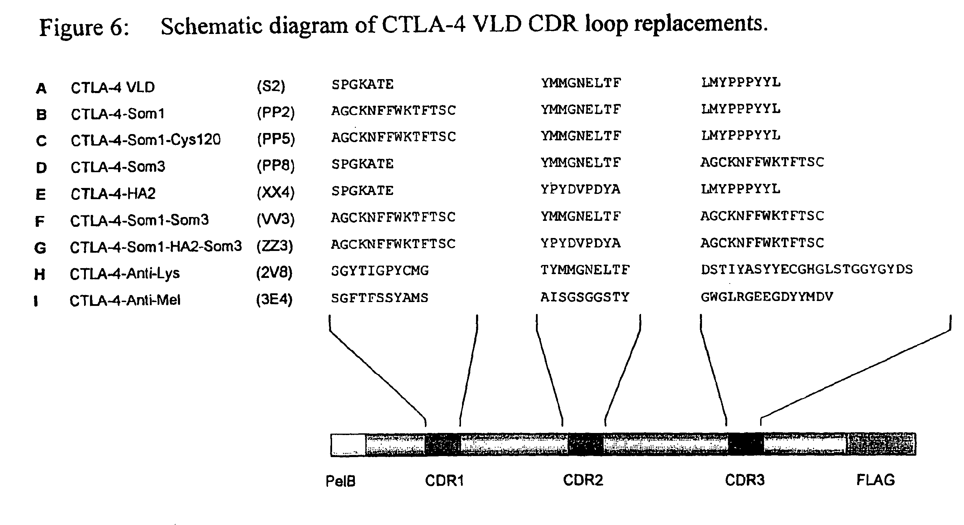

Figure 6: Schematic diagram of CTLA-4 VLD loop replacements. Construct A (CTLA-4 VLD: S2) represents the wild-type CTLA-4 extracellular V-domain, spanning residues 1-115. Constructs B (CTLA-4-Som1; PP2) and C (CTLA-4-Som1-Cys120; PP5) both contain the 14 residue somatostain polypeptide in CDR1. PP5 also carries a C-terminal extension containing Cys120. Construct D (CTLA-4-Som3; PP8) contains the 14 residue somatostatin polypeptide in place of CDR3. In construct E (CTLA-4-HA2: XX4), CDR2 has been replaced with a haemagglutinin tag. In construct F (CTLA-4-Som1-Som3: VV3), both CDR1 and CDR3 have been replaced with the somatostain polypeptide. In construct G (CTLA-4-Som-HA2-Som3: ZZ3) CDR1 and CDR3 are replaced with the somatostatin polypeptide whilst CDR2 is replaced with a haemagglutinin tag. In construct H (CTLA-4-anti-lys:2V8), all three CDR loop structures have been replaced with the CDR loops from a camel anti-lysozyme V_{H}H molecule. Construct I (CTLA-4-anti-mel: 3E4) represents CTLA-4 VLD in which all three CDRs have been replaced by the VH CDR loops from anti-melanoma antibody V86 (Cai And Garen, 1997). PelB, cleavable pectate lyase secretion sequence (22 aa); flag, dual flag tag (AAADYKDDDDKAADYKDDDDK).

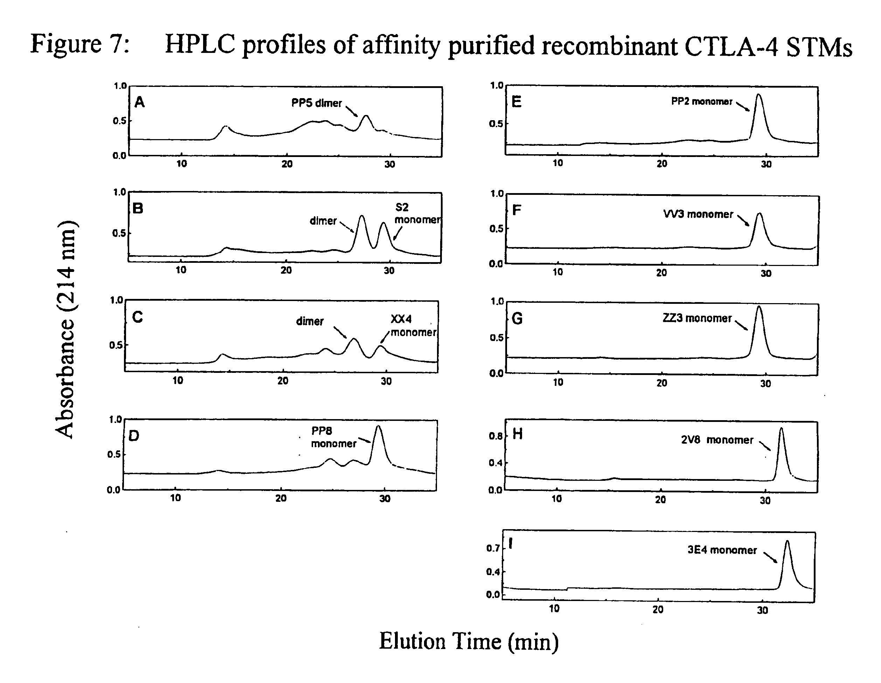

Figure 7: HPLC profiles of purified recombinant human CTLA-4 STMs. Recombinant CTLA-4 VLDs were expressed in E. coli host TG1 from vector pGC, purified from periplasmic extracts by anti-FLAG affinity chromatography and subjected to size exclusion chromatography on a calibrated Superose 12 HR column. The elution profiles of the purified proteins are shown. Panel A, CTLA-4 DIMER (PP5); Panel B, CTLA-4R (S2); Panel C, CTLA-4-HA2 (XX4); Panel D, CTLA-4-Som3 (PP8); Panel E, CTLA-4-Som1 (PP2); Panel F, CTLA-4-Som1-Som3 (W3); Panel G, CTLA-4-Som-HA2-Som3 (ZZ3); Panel H, CTLA-4-anti-lys (2V8); Panel I, CTLA-4-anti-mel (3E4).). Traces represent absorbance at 214 nm and are given in arbitrary units.

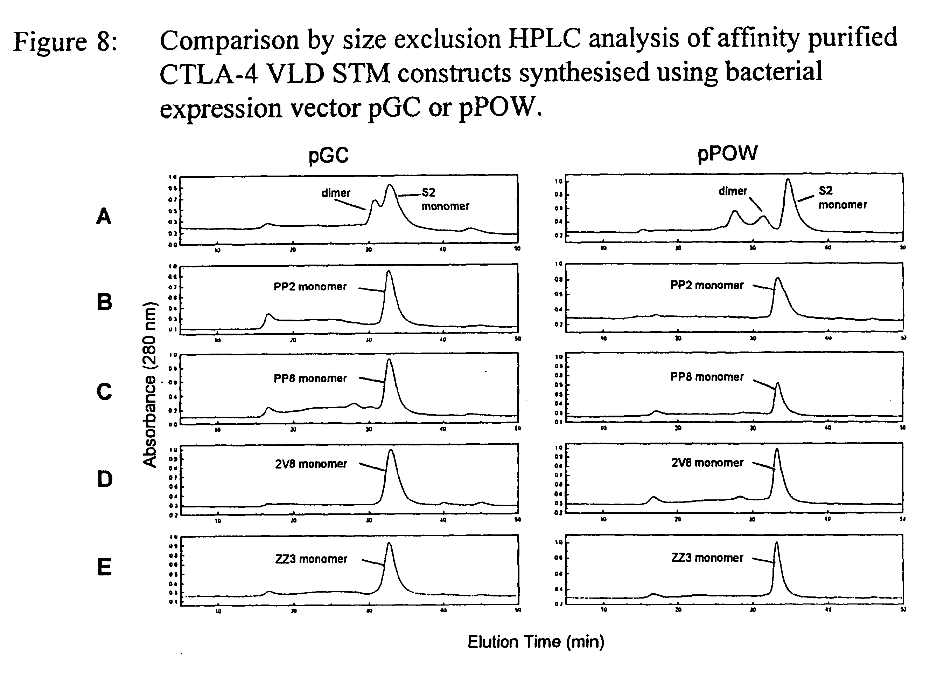

Figure 8: Comparison by size exclusion FPLC analysis of affinity purified CTLA-4 constructs synthesised using bacterial expression vector pGC or pPOW. Recombinant human CTLA-4R or its loop variants were expressed in E. coli host TOP10F', purified from periplasmic extracts by anti-FLAG affinity chromatography and subjected to size exclusion chromatography on a calibrated Superose 12 HR column. The elution profiles of proteins expressed from vector pGC are shown on the left, whilst proteins expressed from vector pPOW are shown on the right. Panel A, wild-type CTLA-4 VLD (S2); B, CTLA-4-Som1(PP2); C, CTLA-4-Som3(PP8); D, CTLA-4-Antilys(2V8); E, CTLA-4-Som1-HA2-Som3(ZZ3).

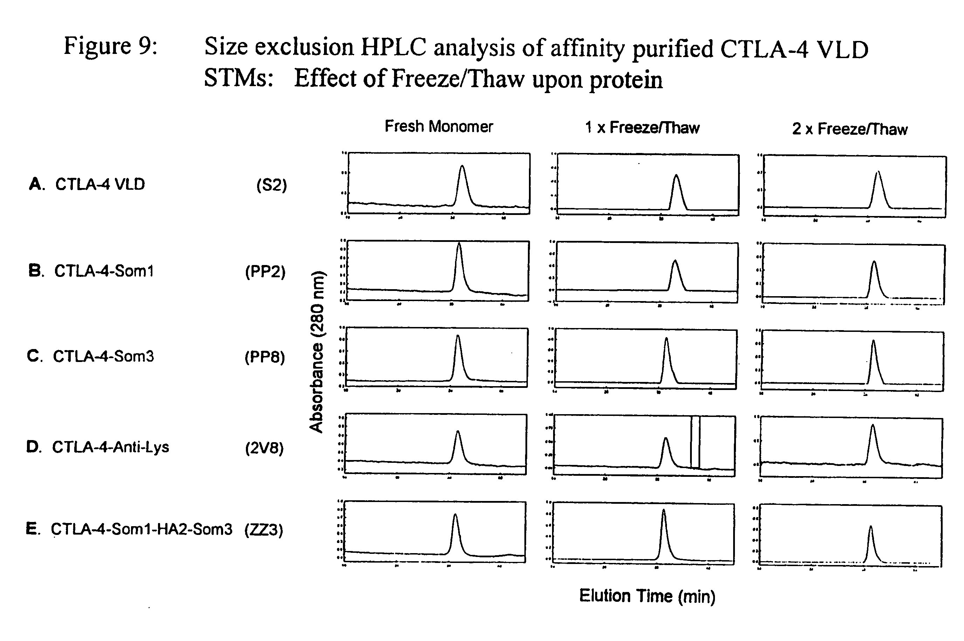

Figure 9: Protein stability analysis. Stability of monomer preparations of CTLA-4 VLD and loop variant constructs was analysed by size exclusion fplc chromatography on a precalibrated superose 12 hr (Pharmacia) column following several cycles of freeze/thawing. Aliquots of each protein were tested immediately after peak purification and following two cycles of freeze/thawing. A, CTLA-4 VLD (S2); B, CTLA-4-Som1 (PP2); C, CTLA-4-Som3 (PP8); D, CTLA-4-anti-lys (2V8); E, CTLA-4-Som-HA2-Som3 (ZZ3).

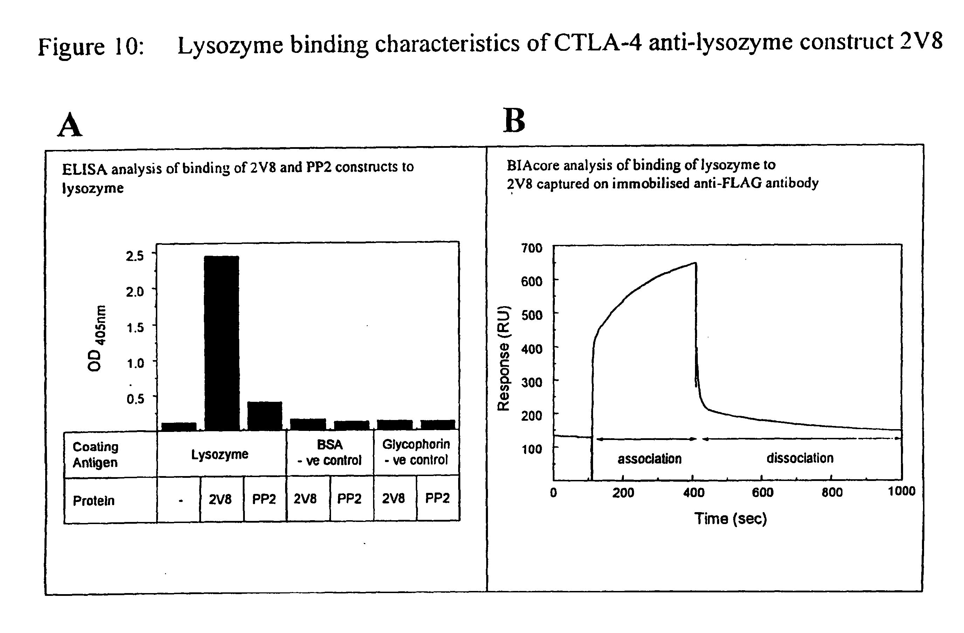

Figure 10: Lysozyme binding characteristics of CTLA-4-anti-lys construct 2V8.

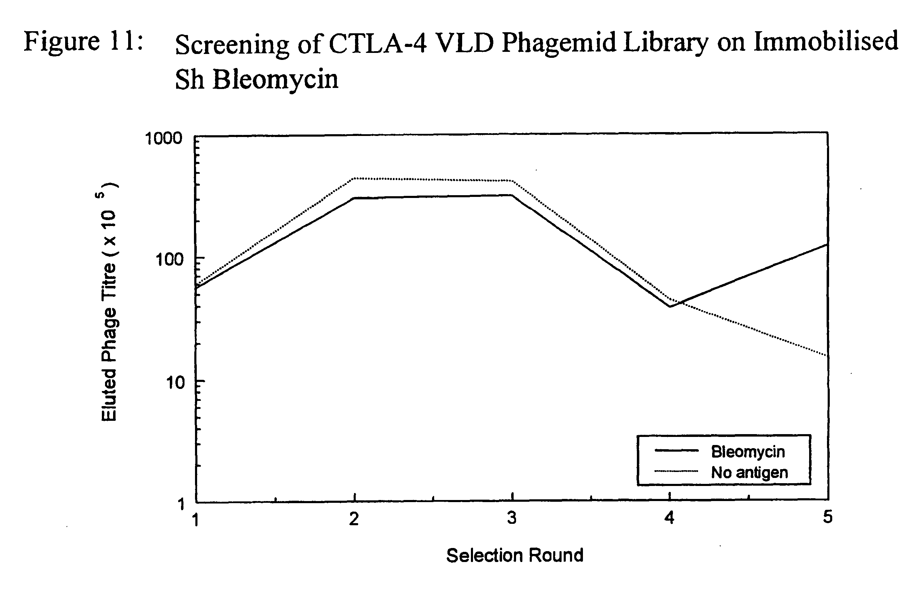

Figure 11: Screening of CTLA-4 VLD phagemid library on immobilised Sh bleomycin.

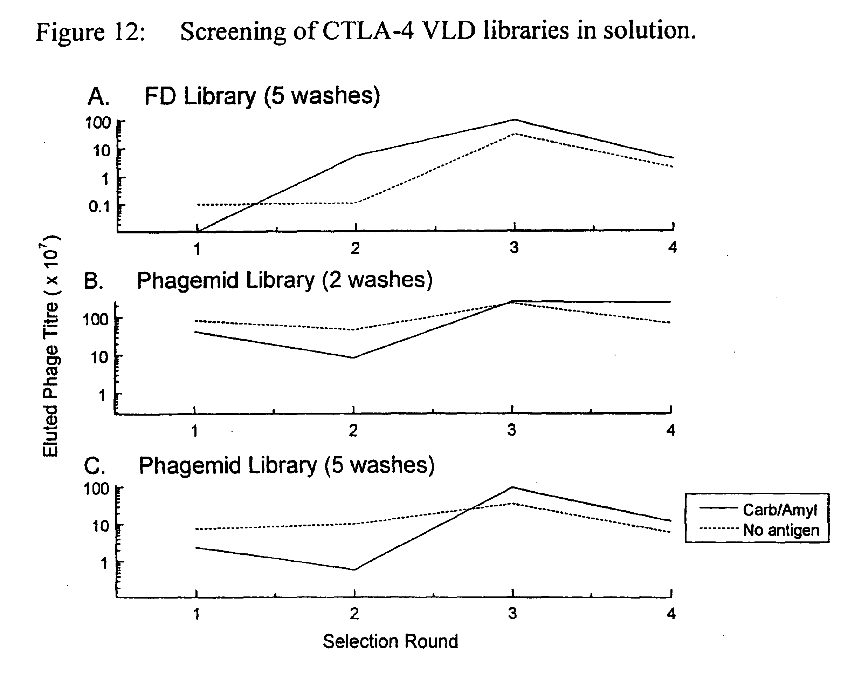

Figure 12: Screening of CTLA-4 VLD libraries in solution

### Detailed Description of the Invention

The present invention relates to the design of novel soluble VLD binding molecules derived from the V-like domain of immunoglobulin superfamily members, such as the human CTLA-4 molecule. The preferred binding molecules of the present invention provide the following advantages (i) use of a native human protein obviates the need for subsequent humanisation of the recombinant molecule, a step often required to protect against immune system response if used in human treatment; (ii) the domain is naturally monomeric as described above (incorporation of residue Cys120 in a C-terminal tail results in production of a dimeric molecule); and (iii) structural modifications have resulted in improved E.coli expression levels.

Prior to publication of the first CTLA-4 structure determination, available sequence data and mutational analyses of both this molecule and CD28 were analysed. This allowed modelling and prediction of the regions corresponding to antibody CDR1, 2 and 3 regions. It was hypothesised that such areas would be susceptible to mutation or substitution without substantial effect upon the molecular framework and hence would allow expression of a correctly folded molecule. The subsequently published structure (Metzler et al. 1997) showed these predictions to be accurate, despite the unexpected separation of CDR1 from the ligand-binding site, and the extensive bending of CDR3 to form a planar surface contiguous with the ligand binding face.

In an initial set of experiments the V-like domain of the human CTLA-4 molecule was modified by replacement of CDR loop structures with either of two defined polypeptides. The two polypeptides were human somatostatin (Som) and a portion of the human influenza virus haemagglutinin protein (HA-tag). Somatostatin (SRIF: somatotropin release-inhibiting factor) is a 14 residue polypeptide comprising a disulphide bond that forces the central 10 residues into a loop. Human somatostatin is biologically widespread within the body and mediates a number of diverse physiological functions such as regulation of growth hormone secretion etc (Reisne, 1995). Human somatostatin binds a number of specific receptors (there are at least five subtypes) which have differing tissue specificities and affinities (Schonbrunn et al. 1995). These aspects of binding and activation are as yet poorly understood, but one salient feature is the high density of somatostatin receptors present on a number of cancerous cell lines, for example cancers of the neuro-endocrine system and small lung cancers (Reubi 1997). Artificial analogues of somatostatin have been produced for imaging of such tumours which are resistant to degradation compared with the highly labile somatostatin polypeptide.

The haemagglutinin epitope sequence consists of the 9 residues YPYDVPDYA. A commercially produced antibody is available which specifically recognises this sequence. The epitope tag can be detected when randomly or directionally incorporated within the structure of proteins (Canfield et al. 1996).

Replacement of one or more CDR loop structures in the CTLA-4 V-like domain with somatostatin or the HA-tag resulted in the production of soluble, monomeric, unglycosylated binding molecules using different bacterial expression systems. This surprising finding shows that V-like domains provide a basic framework for constructing soluble, single domain molecules, where the binding specificity of the molecule may be engineered by modification of the CDR loop structures.

The basic framework residues of the V-like domain may be modified in accordance with structural features present in camelid antibodies. The camel heavy chain immunoglobulins differ from "conventional" antibody structures by consisting of only a single VH domain (Hamers-Casterman et al. 1993). Several unique features allow these antibodies to overcome the dual problems of solubility and inability to present a sufficiently large antigen binding surface.

First, several non-conventional substitutions (predominantly hydrophobic to polar in nature) at exposed framework residues reduce the hydrophobic surface, while maintaining the internal beta-sheet framework structure (Desmyter et al. 1996). Further, within the three CDR loops several structural features compensate for the loss of antigen binding-surface usually provided by the VL domain. While the CDR2 loop does not differ extensively from other VH domains, the CDR-1 and -3 loops adopt non-canonical conformations which are extremely heterologous in length. For example, the H1 loop may contain anywhere between 2-8 residues compared to the usual five in Ig molecules. However, it is the CDR3 loop which exhibits greatest variation: in 17 camel antibody sequences reported, the length of this region varies between 7 and 21 residues (Muyldermans et al. 1994). Thirdly, many camelid VH domains possess a disulphide linkage interconnecting CDRs -1 and -3 in the case of camels and interconnecting CDRs -1 and -2 in the case of llamas (Vu et al. 1997). The function of this structural feature appears to be maintenance of loop stability and providing a more contoured, as distinct from planar, loop conformation which both allows binding to pockets within the antigen and gives an increased surface area. However, not all camelid antibodies possess this disulphide bond suggesting that it is not an absolute structural requirement.

These foregoing features have enabled camelid V-domains to present as soluble molecules in vivo and with sufficiently high affinity to form an effective immune response against a wide variety of target antigens. In contrast, cell surface receptors of the Ig superfamily (such as CD4 and CD2) comprise V-like binding domains and appear to bind cognate receptors with surface features other than the CDR loops. These V-like domains bind to cognate receptors with very low affinity. Physiological signalling between two cells are mediated by the avidity of multi-point binding, when two cell surfaces connect and each contains multiple receptors. CD2 is a well-characterised example: binding to CD58 is mediated by a highly constrained set of minor electrostatic interactions generated by charged and polar residues located in the GFCC'C" face (not the antibody type CDR-1, CDR-2 or CDR-3 loops). This results in a low affinity but highly specific interaction (Bodian et al 1994).

The present invention also relates to a method for generating and selecting single VLD molecules with novel binding affinities for target molecules. This method involves the application of well known molecular evolution techniques to V-like domains derived from members of the immunoglobulin superfamily. The method may involve the production of phage or ribosomal display libraries for screening large numbers of mutated V-like domains.

Filamentous fd-bacteriophage genomes are engineered such that the phage display, on their surface, proteins such as the Ig-like proteins (scFv, Fabs) which are encoded by the DNA that is contained within the phage (Smith, 1985; Huse et al., 1989; McCafferty et al., 1990; Hoogenboom et al., 1991). Protein molecules can be displayed on the surface of Fd bacteriophage, covalently coupled to phage coat proteins encoded by gene III, or less commonly gene VIII. Insertion of antibody genes into the gene III coat protein gives expression of 3-5 recombinant protein molecules per phage, situated at the ends. In contrast, insertion of antibody genes into gene VIII has the potential to display about 2000 copies of the recombinant protein per phage particle, however this is a multivalent system which could mask the affinity of a single displayed protein. Fd phagemid vectors are also used, since they can be easily switched from the display of functional Ig-like fragments on the surface of Fd-bacteriophage to secreting soluble Ig-like fragments in E. coli. Phage-displayed recombinant protein fusions with the N-terminus of the gene III coat protein are made possible by an amber codon strategically positioned between the two protein genes. In amber suppressor strains of E. coli, the resulting Ig domain-gene III fusions become anchored in the phage coat.

A selection process based on protein affinity can be applied to any high-affinity binding reagents such as antibodies, antigens, receptors and ligands (see, for example, Winter and Milstein, 1991, the entire contents of which are incorporated herein by reference). Thus the selection of the highest affinity binding protein displayed on bacteriophage is coupled to the recovery of the gene encoding that protein. Ig-displaying phage can be affinity selected by binding to cognate binding partners covalently coupled to beads or adsorbed to plastic surfaces in a manner similar to ELISA or solid phase radioimmunoassays. While almost any plastic surface will adsorb protein antigens, some commercial products are especially formulated for this purpose, such as Nunc Immunotubes.

Ribosomal display libraries involve polypeptides synthesised de novo in cell-free translation systems and displayed on the surface of ribosomes for selection purposes (Hanes and Pluckthun, 1997; He and Taussig, 1997). The "cell-free translation system" comprises ribosomes, soluble enzymes required for protein synthesis (usually from the same cell as the ribosomes), transfer RNAs, adenosine triphosphate, guanosine triphosphate, a ribonucleoside triphosphate regenerating system (such as phosphoenol pyruvate and pyruvate kinase), and the salts and buffer required to synthesize a protein encoded by an exogenous mRNA. The translation of polypeptides can be made to occur under conditions which maintain intact polysomes, i.e. where ribosomes, mRNA molecule and translated polypeptides are associated in a single complex. This effectively leads to "ribosome display" of the translated polypeptide.

For selection, the translated polypeptides, in association with the corresponding ribosome complex, are mixed with a target molecule which is bound to a matrix (e.g. Dynabeads). The target molecule may be any compound of interest (or a portion thereof) such as a DNA molecule, a protein, a receptor, a cell surface molecule, a metabolite, an antibody, a hormone or a virus. The ribosomes displaying the translated polypeptides will bind the target molecule and these complexes can be selected and the mRNA re-amplified using RT-PCR.

Although there are several alternative approaches to modify binding molecules the general approach for all displayed proteins conforms to a pattern in which individual binding reagents are selected from display libraries by affinity to their cognate receptor. The genes encoding these reagents are modified by any one or combination of a number of *in vivo* and *in vitro* mutation strategies and constructed as a new gene pool for display and selection of the highest affinity binding molecules.

In order that the nature of the present invention may be more clearly understood preferred forms thereof will now be described with reference to the following examples.

### Example 1

### Gene Construction and Cloning

CTLA-4 STM (STM: soluble truncated mutants of CTLA-4, used herein to describe CTLA-4 chimaeric V-like domain proteins) gene construction and cloning was by standard and well-described techniques (Polymerase chain reaction with specifically designed oligonucleotide primers, splice overlap extension, restriction enzyme digests etc). A list of oligonucleotide primers used is given in Figure 1.

The wild-type STM construct was amplified from cloned human CTLA-4 DNA (Figure 2) (and could be similarly amplified from reverse transcribed human cDNA by a competent worker in the field) using the oligonucleotide primers #3553 and #4316, which incorporated SfiI and NotI restriction sites at the 5' and 3' ends respectively. These terminal primers were used in all further constructions except: (i) where #4851 or #5443 was used to incorporate an ApaL1 site at the 5' end; (ii) where #4486 was used to add a C-terminal tail including residue Cys120; (iii) where #5467 was used to incorporate an EcoR1 site at the 5' end; and (iv) where the specific set of extension primers were used for ribosomal display.

A splice overlap PCR strategy using combinations of the oligonucleotides primers listed in Figure 1 was used to produce variations of CDR-1, CDR-2 and/or CDR-3 loop structure replacements. The variations, which are described in greater detail in the following examples are listed in Table 2.

**TABLE 2**

| ***CDR-1 combinations*** | | |
|---|---|---|
| **CDR-1** | | |
| CTLA-4 VLD | S¹⁹PVCEYA.**SPGKATE**..... | VRV... |
| | | |
| Anti-lysozyme | S¹⁹FVCEYA.**SGYTIGPYCMG.....** | VRV... |
| Somatostatin-14 | S¹⁹FVCEYA.**AGCKNFFWKTFTSCATE**. | VRV... |
| Anti-melanoma | S¹⁹FVCEYA.**SGFTFSSYAMS.....** | VRV... |
| | | |
| Randomisation 1 | S¹⁹FVCEYA.**XXXXXXXG.....** | VRV... |
| Randomisation 2 | S¹⁹FVCEYA.**XXXXXXXXCXG.....** | VRV... |
| Randomisation 3 | S¹⁹FVCEYA.**XXarXarXXarCXG.....** | VRV... |
| Randomisation 4 | S¹⁹FVCEYA.**SPGXXXX.....** | VRV... |
| Randomisation 5 | S¹⁹FVCEYA.**SPGXCXX.....** | VRV... |
| Randomisation 6 | S¹⁹FVCEYA.**XXXXXXXXATE.....** | VRV... |
| Randomisation 7 | S¹⁹FVCEYA.**XXXXXXCXATE.....** | VRV... |
| Randomisation 8 | S¹⁹FVCEYA.**AGCKNXXXXXXTSCATE.** | VRV... |
| | | |

| ***CDR-2 combinations*** | | |
|---|---|---|
| **CDR-2** | | |
| CTLA-4 VLD | Q⁴⁴VTEVCAA.**TYMMGNELTF.**LDDSICT... | |
| | | |
| Anti-lysozyme | Q⁴⁴VTEVCAA.**AINMGGGITF.**LDDSICT... | |
| Haemagglutinin tag | Q⁴⁴VTEVCAA.**TYPYDVPDYA.**LDDSICT... | |
| Anti-melanoma | Q⁴⁴VTEVCAA.**AISGSGGSTY.**LDDSICT... | |
| | Q⁴⁴VTEVCAA.**TYXXGXELTF**.LDDSICT... | |
| Randomisation 1 | | |
| Randomisation 2 | Q⁴⁴VTEVCAA.**CYXXGXELTF**.LDDSICT... | |

| ***CDR-3 combinations*** | | |
|---|---|---|
| **CDR-3** | | |
| CTLA-4 VLD | C⁹³KV.**ELMYPPPYYL**..... | GIG... |
| | | |
| Anti-lysozyme | C⁹³KV.**DSTIYASYYECGHGLSTGGYGYDS.** | GIG... |
| Somatostatin-14 | C⁹³KV.**EAGCKNFFWKTFTSC.....** | GIG... |
| Anti-melanoma | C⁹³KV.**GWGLRGEEGDYYMDV.....** | GIG... |
| | | |
| Randomisation 1 | C⁹³KV.**XXXXXXXXXXX.....** | GIG... |
| Randomisation 2 | C⁹³KV.**XXXXXXXXXXXXXXX.....** | GIG... |
| Randomisation 3 | C⁹³KV.**XXXXXX.C.XXXX.....** | GIG... |
| Randomisation 4 | C⁹³KV.**XXXXXXX.C.XXXX.....** | GIG... |
| Randomisation 5 | C⁹³KV.**XXXXXXXX.C.XXXXX.....** | GIG... |
| Randomisation 6 | C⁹³KV.**XXXXXXXXXX.C.XXXXXXX.....** | GIG... |
| | | |
| Randomisation 7 | C⁹³KV.**EXXXXXXXXX.....** | GIG... |
| Randomisation 8 | C⁹³KV.**EXXXXXX.C.XXXXXXX.....** | GIG... |
| Randomisation 9 | C⁹³KV.**EAGCKNXXXXXXTSC.....** | GIG... |

For generation of randomised sections of the CDR loop structures, similar splice-overlap techniques were used with oligonucleotides where a given triplet(s) were encoded by the sequence NNg/T where N represents any of the four possible nucleotide bases. This combination covers all possible amino acid residues. Alternatively, randomisation was biased towards certain subsets of amino acids (for example aromatic residues, Figure 1, #5452).

In some instances, a variant technique was used for STM gene construction, where randomised oligonucleotide primers were designed which incorporated restriction sites for direct cloning into the similarly modified (with complementary restriction sites) CTLA-4 VLD framework (for example Figure 1, #4254).

Completed constructs were cut with approriate combinations of restriction enzymes (for example Sfi1,Not1, ApaL1, EcoR1) and cloned into like sites in appropriate expression vectors. These vectors comprise: (i) for production of soluble protein expression vectors pGC (Coia et al, 1996) and pPOW (Power et al, 1992; Kortt et al. 1995) (ii) for bacteriophage and phagemid display, completed STM constructs were cut with the restriction enzymes SfiI and NotI or ApaLI and NotI and cloned into the vectors pHFA, and pFAB.5c (phagemid) or pfd-Tet-DOG (phage). These vectors allow display of the STMs as gene3 protein fusions on the surface of bacteriophage in 1-2 (phagemid) or 3-5 (phage) copies per bacteriophage particle (Figure 3).

All DNA constructs were verified by restriction analysis and DNA sequencing and tested for expression of recombinant protein by standard and well-understood techniques (Polyacrylamide gel electrophoresis, Western blot etc).

### Example 2

### Production and Isolation of Recombinant STM Proteins

Recombinant proteins were produced using vectors which represent different protocols for periplasmic expression systems. These vectors were (i) pGC: this vector allows high level expression of heterologous proteins by chemical (IPTG) induction, which are targeted to the periplasmic space by means of a leader sequence. The leader sequence is subsequently cleaved to produce the mature protein. In addition, this vector contains two in-frame 8 residue tag sequences (FLAG tags) which allow affinity purification of the recombinant protein. (ii) pPOW, which, like pGC, allows high level heat inducible expression of proteins targeted to the periplasmic space by means of a cleavable leader sequence and two in-frame 8 residue tag sequences (FLAG tags).

Recombinant proteins were purified by the following methods, which are but two variations of well established techniques. (i) Bacterial clones in vector pGC were grown overnight in 2YT medium/37°C /200 rpm/100mg/ml ampicillin, 1% glucose (final). Bacteria were diluted 1/100 into either 0.5 or 21 of 2YT medium supplemented with 100mg/ml ampicillin, 0.1% glucose (final), and grown at 28°C/ 200 rpm. These cultures were grown to an optical density of between 0.2-0.4, at which stage they were induced with 1mM IPTG (final). Cultures were allowed to grow for 16 hours (overnight) before harvesting. Bacteria were collected by centrifugation (Beckman JA-14 rotor or equivalent/6K/10min/4°C) and the periplasmic fraction collected by standard techniques. Briefly, this involved resuspension of bacterial pellets in a 1/25th volume of spheroplast forming buffer consisting of 100mM Tris-HCl/0.5M sucrose/0.5 mM EDTA (pH8.0), followed by addition of 1/500th volume of hen egg lysozyme (2mg/ml in water) and incubation for 10min. A 0.5x solution of the above spheroplasting buffer was then added to a final volume of 1/5th of the original culture, and the incubation continued for a further 20min. The cell debris was then pelleted by centrifugation (Beckman JA-14 rotor or equivalent/9K/15min/4°C) and the supernatant containing the periplasmic fraction collected. All of the above procedures were performed at 4°C. Samples were processed immediately by sonication, filtration through a 0.45µ nitrocellulose membrane and processed immediately or stored at 4°C in the presence of sodium azide (0.05%). If freezing was required, no more than one freeze-thaw cycle was allowed. (ii) Bacterial clones in pPOW were grown overnight at 30°C in 100 ml 2xYT broth containing 100 µg/ml (w/v) ampicillin. On the following day cultures were used to inoculate 900 ml fresh 2xYT broth containing 100 µg/ml (w/v) ampicillin, to OD600nm = 0.2-0.5, and grown at 30°C with shaking (150-200 rpm) until OD600nm = 4 i.e. late log phase. To induce recombinant protein expression, the temperature was raised to 42°C for 1 hour and then dropped to 20°C for a further hour. Cells were harvested by centrifugation (Beckman JA-14 /6K rpm/5 min/4°C), the cell pellet resuspended in 100 ml extraction buffer (20mM Tris pH 8.0/ 0.2mg/ml (w/v) lysozyme/0.1% (v/v) Tween-20) and incubated at 4°C overnight. Samples were sonicated for 30 seconds and cellular debris collected by centrifugation (Beckman JA-14 /14K rpm/10 min/4°C). The aqueous phase, containing the "lysozyme" wash, was retained. Cell pellets were then washed twice with ice-cold water and this "osmotic shock" wash was retained. Each wash consisted of resuspending the pellet in 100 ml ice-cold water followed by incubation on ice for 10 minutes in the first instance followed by 1 hour in the second instance. Following centrifugation (Beckman JA-14 /14K rpm/10 min/4°C), the pH of the aqueous phase was adjusted by addition of 10 ml 10xTBS, pH 8. The "lysozyme" and "osmotic shock" washes were pooled and constitute the soluble or "periplasmic" protein fraction. Periplasmic fractions were sonicated, filtered through a 0.45µ nitrocellulose membrane and processed immediately or stored at 4°C in the presence of sodium azide (0.05%), PMSF (23 µg/ml) and EDTA (50 mM).

Recombinant proteins were purified by affinity chromatography through a divinyl sulphone activated agarose (Mini-Leak)-linked anti-FLAG antibody column. Periplasmic extracts were directly loaded onto a 10 ml anti-FLAG column which had been pre-equilibrated in TBS (pH 8) containing 0.05% (w/v) sodium azide. Bound proteins were eluted with Immunopure Gentle Ag/Ab Elution Buffer (Pierce). Samples were then dialysed against TBS/0.05% (w/v) azide (pH 8), concentrated by ultrafiltration over a 3 kDa cut-off membrane (YM3, Diaflo), and analysed by HPLC on a pre-calibrated Superose 12 HR or Superdex 200 HR column (Pharmacia Biotech), at a flow rate of 0.5 ml/min. Fractions corresponding to monomeric , dimeric and tetrameric species were collected, concentrated as above, and stored at 4°C prior to analysis. Protein concentration was determined spectrophotometrically using an extinction coefficient at A280 of 1.27 for the CTLA-4R extracellular domain, 0.92 for CTLA-4-Som1, 1.13 for CTLA-4-Som3, 1.05 for CTLA-4-Anti-Lys. All of the above protein chemistry methods are standard techniques within the field. Purified proteins were analysed by standard techniques for example polyacrylamide gel electrophoresis, western blot, dot blot etc.

Cloning and expression in the bacteriophage expression vectors pHFA, pFAB.5c and fd-tet dog, and subsequent production of recombinant bacteriophage, were by standard and well-established techniques. Screening of libraries of randomised CTLA-4 STMs was by standard and well-established techniques (Galanis et al 1997).

### Example 3

### CTLA-4 STMs incorporating Somatostatin and Haemagglutinin Peptides.

Initially the CDR1 or the CDR3 loop structures of the CTLA-4 STM were replaced with the somatostatin polypeptide. This 14 residue polypeptide is conformationally constrained by an intra-disulphide linkage between Cys3 and Cys14 (Figure 4). This was reasoned to form a discrete protein loop, analogous to the CDR loops found in antibodies, and particularly analogous to the long CDRs found in camelid antibodies which are also stabilised by a disulphide linkage. The effect of substituting CDR1 in the presence or absence of Cys120 ie. whether a dimer could be produced, was also tested. These experiments produced an unexpected and surprising result. Substitution of either CDR-1 or -3 with somatostatin significantly enhanced the production of monomeric protein. This is illustrated in Figure 5 where replacement of the CDR-3 loop structure with somatostatin significantly increased the ratio of monomeric to dimeric/tetrameric protein species.

In further experiments, simultaneous replacement of both CDR-1 and-3 loop structures by somatostatin resulted in production of high-levels of monomeric protein. This shows that extensive loop structure replacements can be accommodated by the CTLA-4 scaffolding. Structurally, one of the somatostatin loops may lie flat against the face of the molecule in a manner analogous to that of the CDR-3 loop structure of CTLA-4 VLD.

In a further extension of the CDR loop structure-replacement strategy, a region corresponding to CDR-2 was replaced with the 8-residue haemagglutinin (HA) tag sequence. Use of the conformationally constrained somatostatin loop in this position was considered unsuitable as this region partially encompasses the C" beta strand running the length of the molecule. The HA tag could be detected upon this CTLA-4 STM by use of an anti-HA antibody. Gel filtration experiments showed the presence of a range of protein species, from monomeric through to aggregated species suggesting that CDR-2-only substitutions were not stable (Figures 6,7).

Final proof of principle that the CTLA-4 CDR loop structures could be replaced with other polypeptides to produce monomeric, soluble, STMs was by simultaneous replacement of all three CDR loop structures with two somatostatin and one HA epitope respectively. This STM produced a correctly folded and monomeric protein upon gel filtration chromatography (Figures 6,7).

The positions of CDR loop structure substitutions within the CTLA-4 VLD for the various STMs are shown in figure 6. HPLC profiles of affinity-purified STM proteins are shown in figure 7. Identical results were obtained for proteins produced in two different protein expression systems: pGC where protein expression is chemically induced, and pPOW where protein expression is temperature induced (see Example 2)(Figure 8). Polyacrylamide gel electrophoresis followed by western blot analysis indicated that the CTLA-4 STMs could be reduced and ran at the expected molecular weights and absent of glycosylation. Testing of isolated monomeric STM proteins showed that they remained monomeric after zero, one, or two freeze-thaw cycles (figure 9).

CTLA-4 STMs retained the correct conformation since a conformationally-specific anti-CTLA-4 antibody recognised STMs with both CDR1 and -3 loop structure replacements. Interestingly, this antibody recognised the wild type monomer and the dimer (CDR1 replaced) poorly, contrasting with the strong reaction observed for the modified protein species. This suggests that in the wild type STM some form of local interaction is occurring that occludes the antibody binding site, and that this interaction is similar to the result when two CTLA-4 molecules are tethered together (presumably blocking access to the antibody).

### Example 4

### CTLA-4 STMs Based Upon a Camel anti-Lysozyme Antibody.

The camel V_{H}H antibody cAb-Lys3 isolated from immunised camels specifically binds within the active site cleft of hen egg lysozyme (Desmyter et al. 1996). To illustrate the ability of CTLA-4 STMs to function in a similar fashion, the three CDR loop structures of CTLA-4 VLD STM were replaced with the three CDR loop regions from cAb-Lys3. Positions and sequence of the substitutions are shown in figure 6. Expression of this STM (2V8) in either pGC or pPOW based expression systems resulted in production of predominantly monomeric soluble protein (Figures 7, 8). Protein solubility of this CTLA-4 STM was superior to native CTLA-4 VLD. ELISA analysis showed that (pGC produced) purified monomeric protein specifically bound hen egg lysozyme compared to non-specific antigens and compared to the CTLA-4 STM with somatostatin substituted within the CDR1 loop structure (PP2) (Figure 10A). Real-time binding analysis by BIAcore showed that the lysozyme specifically bound to immobilised anti-lysozyme STM (Figure 10B). The CTLA-4 STM framework is thus folding correctly and presenting the CDR loop structures in a manner in which they can bind lysozyme antigen. To further enhance expression of the CTLA-4 VLD anti-lysozyme, the coding sequence was adjusted by splice overlap PCR to comprise codons preferential for *E. coli* expression.

### Example 5

### CTLA-4 STMs Based Upon a Human anti-Melanoma Antibody.

The human-derived anti-melanoma antibody V86 specifically binds human melanoma cells. This antibody is unusual in that binding affinity resides entirely within the V_{H} region, addition of a cognate V_{L} decreases binding efficiency, and that the V_{H} domain expressed with a small segment of the V_{L} domain has a high degree of solubility (Cai and Garen, 1997). To further illustrate that replacement of CTLA-4 VLD CDR loop structures enhances solubility and that the resultant STMs can be produced in bacterial expression systems, the three CDR loop structures of CTLA-4 were replaced with the three CDR loop regions from V86. Positions and sequence of the substitutions are shown in Figure 6. Expression of this STM (3E4) in pGC again resulted in production of predominantly monomeric soluble protein (Figure 7) with enhanced solubility compared to the CTLA-4 VLD.

### Example 6

### Construction of CTLA-4 STMs as Libraries of Binding Molecules

To select CTLA-4 STMs with novel binding specificities, VLD libraries were produced containing randomised CDR1 and CDR3 loop structures. Oligonucleotide primers used for library construction are listed in figure 1. Combinations of oligonucleotide primers used for library construction are shown in Table 3.

**Table 3. CTLA-4 STM Library Combinations**

| ***CDR1*** | | | | | | | | |
|---|---|---|---|---|---|---|---|---|
| CDR3 | 4483* | 4254 | 5449 | 5451 | 5452 | 5450 | 5446 | 4835 |
| 4482 | + 1 | + 1 | ////////// | ////////// | ////////// | ////////// | ////////// | ////////// |
| 4275 | + 1 | + 1 | ////////// | ////////// | ////////// | ////////// | ////////// | ////////// |
| 5470 | ////////// | ////////// | + 2 | + 2 | + 2 | + 2 | + 2 | ////////// |
| 5474 | ////////// | ////////// | + 2 | + 2 | + 2 | + 2 | + 2 | ////////// |
| 5471 | ////////// | ////////// | + 2 | + 2 | + 2 | + 2 | + 2 | ////////// |
| 5472 | ////////// | ////////// | + 2 | + 2 | + 2 | + 2 | + 2 | ////////// |
| 5475 | ////////// | ////////// | + 2 | + 2 | + 2 | + 2 | + 2 | ////////// |
| 5473 | ////////// | ////////// | + 2 | + 2 | + 2 | + 2 | + 2 | ////////// |
| 4836 | ////////// | ////////// | ////////// | ////////// | ////////// | ////////// | ////////// | + 3 |

| | | | | | | | | |
|---|---|---|---|---|---|---|---|---|
| *: oligonucleotide number. + : library combination. 1,2,3: describes library number. | | | | | | | | |

DNA constructs encoding the resultant libraries were cloned into vectors pHFA or pFAB.5c for production of fd-phagemid based libraries and into pfd-Tet-Dog for production of fd-phage based libraries (see examples 1 and 2). Library 1 was cloned into vector pHFA and consisted of 2.1 x 10⁷ independent clones. Library 3 was cloned into vectors pHFA (5.7 x 10⁵ independent clones) and pfd-Tet-Dog (2.2 x 10⁴ independent clones). Library 2 was cloned into pFAB.5c (1.7 x 10⁷ independent clones) and into pfd-Tet-Dog (1.6 x 10⁵ independent clones). Numbers of independent clones were determined by counting gross numbers of transformed colonies constituting the library, followed by assaying for the presence and proportion of CTLA-4 STM-specific DNA.

For library 2, the variability of the full library was tested by sequencing of representative clones. These results are presented in Table 4. The expected heterogeneity of insert size and sequence was observed. A high proportion of UAG termination codons were observed, consistent with the oligonucleotide randomisation strategy. To prevent these codons causing premature termination of the CTLA-4 STM gene3 protein fusions, libraries were transferred into the *E.coli* strains Tg-1 and JM109, which suppress this termination codon by insertion of a glutamic acid residue. Cysteine residues were present in the high numbers expected from the desgn of the oligonucleotides,and were in positions capable of forming intra- and inter-CDR loop structure disulphide bonds.

**Table 4 CDR1 and CDR3 Inserts from a Representative Series of Library 2 Clones**

| CLONE | CDR1 | CDR3 |
|---|---|---|
| 3M-2 | ND1 | LPSSDTRAYS |
| 3M-3 | QESGGRPG | LPRGPPLLSL |
| 3M-5 | SPGRCLN | ND |
| 3M-6 | EWKR*HGG | LCPGACGCVY |
| 3M-7 | NSG*NEGG | ND |
| 3M-11 | DKPVTKSG | ND |
| 3M-17 | SPGACP* | ND |
| 3M-18 | SPGKCDQ | ND |
| 3M-19 | SPGMCAR | LMYPPPYYL |
| 3M-20 | ND | PFLFLPC*FFF |
| 3N-1 | WTLGHHKLCEG | LFTCLLALCS |
| 3N-2 | SPGECYG | SWLSTTXCLSSCS |
| 3N-3 | SPG*CQD | LLGSLLSCFASLS |
| 3N-4 | SPG*CQD | SPGSLLSCFASXS |
| 3N-5 | SPGRCTD | VICHSSVCLSD/EVC |
| 3N-6 | ND | DLPSYLACSI |
| 3N-7 | SPGRCDA | ALCWDVFYCSFPSY |
| 3N-8 | ELFGHARYCKG | VSITSP*LCPVKVFD |
| 3N-9 | SPGKV*N | LFVPFVSP |
| 3N-12 | SPGDLWV | ESGLSPVSPCSLYSL |
| 3N-13 | TSANGPYG | PWAYRFLAVL |
| 3N-14 | RKTREKYG | ELMYPPPYYLGI |
| 3N-15 | SPGQELT | ELFFLLYAPCYLFQR |

| | | |
|---|---|---|
| ND: Not Done *: UAG termination codon | | |

Bacteriophage particles displaying CTLA-4 STMs as gene 3 protein fusions were rescued from *E.coli* cells by standard protocols and panned against antigens presented in a number of contexts as described in the following examples.

### Example 7

### CTLA-4 STM Libraries: Selection against Antigens on Solid Supports.

Four different antigens falling into a class of proteins with clefts or crevices within their structures were selected for screening. It was anticipated that the CTLA-4 VLD STMs, being of smaller size than antibodies, and possessing elongated CDR loop structures (especially CDR-3) would be able to access these cleft regions. The antigens selected were: (i) hen egg lysozyme (EC 3.2.1.17); (ii) bovine carbonic anhydrase (EC 4.2.1.1); (iii) fungal a-amylase (EC 3.2.1.1); and (iv) *Streptoalloteichus hindustanis* resistance protein ShBle (Gatignol et al. 1988). For binding to plates, antigens in coating buffer (1mg/ml in 0.1M NaHCO3 pH8.5) were bound to Costar ELISA plates by standard procedures. Rescued phage and phagemid-derived libraries were panned by standard and well-understood procedures except that lower than standard number of washes were employed to allow low affinity binding phage to be selected. Figure 11 shows titres of libraries selected against ShBle. After round 4, recovered bacteriophage titres were higher than controls. To those skilled in the art, this represents selection of specific binding moieties, and it is then a routine process to produce these selected CTLA-4 VLD STMs using expression vectors such as pGC or pPOW (as described in example 2).

### Example 8

### CTLA-4 STM Libraries: Selection against Antigens in Solution.

For selection in solution, the antigens bovine carbonic anhydrase and fungal a-amylase were biotinylated and selections performed in solution using capture by streptavidin coated magnetic beads. Throughout these experiments washes were kept constant at either 2 or 5 washes per selection round. Titres of recovered bacteriophage post-elution are shown in Figure 12. After round 4, recovered bacteriophage titres were higher than controls. To those skilled in the art, this represents selection of specific binding moieties, and it is then a routine process to produce these selected CTLA-4 VLD STMs using expression vectors such as pGC or pPOW (as described in example 2).

### Example 9

### CTLA-4 STM Libraries: Selection in an Alternative Display and Selection System.

To allow further maturation and selection of antigen binding STMs, the CTLA-4 STM library was ligated into a plasmid to add a downstream C-terminal spacer polypeptide (heavy constant domain). Upstream transcriptional and translational initiation sequences were added by PCR amplification using specific oligonucleotides (Figure 1). This PCR DNA was used as a template for production of RNA followed by translation and display of the library on ribosomes in a coupled cell free translation system as described by He and Taussig (1997). To demonstrate binding, CTLA-4 STM ribosome complexes were panned on hepatitis B surface antigen (hbsa), glycophorin (glyA) and bovine serum albumin (BSA) coated dynabeads. RNA from ribosome complexes bound to hbsa, glyA and BSA was recovered by RT-PCR. It is then a routine process to clone these RT-PCR products into an expression vector such as pGC or pPOW (as described in example 2) allowing production of CTLA-4 STMs. It will be appreciated by persons skilled in the art that numerous variations and/or modifications may be made to the invention allowing display of libraries of CTLA-4 STMs as ribosome complexes (as in this example) as well as display on the surface of live cells (which may be derived from a eukaryotic or prokaryotic background) and may include bacteria, yeast, mammalian or insect cells.

### Example 10

### CTLA-4 STMs: Affinity Maturation and CDR2 Mutation.

To allow further maturation and selection of antigen-binding STMs, and the construction of randomised CDR-1, -2 and -3 libraries, CDR-2 randomised oligonucleotide primers were produced (Figure 1). A variation of these primers contained conserved cysteine residues to allow construction of STMs with CDR2-CDR3 disulphide linkages mimicing those found in llama single domain antibodies. Splice overlap PCR allowed creation of libraries containing all three CDR loop structures randomised.

### REFERENCES

Bodian DL, Jones EY, Harlos K, Stuart DI, Davis SJ (1994) Crystal structure of the extracellular region of the human cell adhesion molecule CD2 at 2.5 A resolution. Structure 2: 755-766
Cai X and Garen A (1997) Comparison of fusion phage libraries displaying VH or single-chain Fv antibody fragments derived from the antibody repertoire of a vaccinated melanoma patient as a source of melanoma-specific targeting molecules Immunol 94: 9261-9266
Canfield VA, Norbeck L, Levenson R (1996) Localization of cytoplasmic and extracellular domains of Na,K-ATPase by epitope tag insertion Biochem. 35: 14165-14172
Coia, G., Hudson, P.J., Lilley, G.G. (1996) Construction of recombinant extended single-chain antibody peptide conjugates for use in the diagnosis of HIV-1 and HIV-2. J. Immunol. Meth. 192:13-23
Davies J, Riechmann L (1994) Camelising human antibody fragments: NMR studies on VH domains FEBS Lett. 339(3):285-90
Davies J, Riechmann L. (1996) Single antibody domains as small recognition units: design and in vitro antigen selection of camelized, human VH domains with improved protein stability Protein Eng 9(6):531-7
Desmyter A, Transue TR, Ghahroudi MA, Thi MH, Poortmans F, Hamers R, Muyldermans S, Wyns L (1996) Crystal structure of a camel single-domain VH antibody fragment in complex with lysozyme Nat Struct Biol 3:803-811
Galanis M, Irving RA, and Hudson PJ (1997) Current Protocols in Immunology, 17.1.1-17.1.45.
Gatignol A, Durand H, and Tiraby G (1988) Bleomycin resistance conferred by a drug-binding protein FEBS Lett 230: 171-175
Gerstmayer B, Pessara U, Wels W (1997) Construction and expression in the yeast Pichia pastoris of functionally active soluble forms of the human costimulatory molecules B7-1 and B7-2 and the B7 counter-receptor CTLA-4. FEBS Lett 407: 63-68
Hamers-Casterman C, Atarhouch T, Muyldermans S, Robinson G, Hamers C, Songa EB, Bendahman N, Hamers R (1993) Naturally occurring antibodies devoid of light chains. Nature 363: 446-448
Hanes J and Pluckthun A (1997) In vitro selection and evolution of functional proteins by using ribosome display Proc.Natl.Acad. Sci. USA. 94: 4937-4942
He M and Taussig MJ (1997) Antibody-ribosome-mRNA (ARM) complexes as efficient selection particles for in vitro display and evolution of antibody combining sites. Nucl. Acids Res. 25: 5132-5134
Hoogenboom, H.R., Griffiths, A.D., Johnson, K.S., Chiswell, D.J., Hudson, P., and Winter, G. 1991. Multi-subunit proteins on the surface of filamentous phage: methodologies for displaying antibody (Fab) heavy and light chains. Nucleic Acids Res. 19:4133-4137.
Huse, W. D.,Sastry, L.,Iverson, S. A.,Kang, A. S.,Alting, M. M.,Burton, D. R.,Benkovic, S. J., & Lerner, R. A. (1989). Generation of a large combinatorial library of the immunoglobulin repertoire in phage lambda . Science, 246: 1275-81.
Hutloff, A., Dittrich, A.M., Beier, K.C., Elijaschewitsch, B., Kraft, R., Anagnostopoulos, I. and Kroczek, R.A. (1999). ICOS is an inducible T-cell co-stimulator structurally and functionally related to CD28. Nature 397: 263-266.
Kortt, A.A., Guthrie, R.E., Hinds, M.G., Power, B.E., Ivancic, N., Caldwell, J.B., Gruen, L.C., Norton, R.S. and Hudson, P.J. (1995) Solution properties of E. coli expressed VH domain of anti-neuraminidase antibody NC41. J. Protein Chemistry. 14, 167-178.
Linsley, PS, Clark EA, and Ledbetter JA (1990) The T cell antigen CD28 mediates adhesion with B cells by interacting with the activation antigen, B7/BB1 Proc. Natl. Acad. Sci USA 87: 5031.
Linsley PS, Nadler SG, Bajorath J, Peach R, Leung HT, Rogers J, Bradshaw J, Stebbins M, Leytze G, Brady W, et al (1995) Binding stoichiometry of the cytotoxic T lymphocyte-associated molecule-4 (CTLA-4). A disulfide-linked homodimer binds two CD86 molecules. J Biol Chem 270: 15417-24
McCafferty, J.,Griffiths, A. D.,Winter, G., & Chiswell, D. J. (1990). Phage antibodies: filamentous phage displaying antibody variable domains. Nature, 348: 552-4.
Metzler WJ, Bajorath J, Fenderson W, Shaw SY, Constantine KL, Naemura J, Leytze G, Peach RJ, Lavoie TB, Mueller L, Linsley PS (1997) Solution structure of human CTLA-4 and delineation of a CD80/CD86 binding site conserved in CD28 Nat Struct Biol 4: 527-531
Morton PA, Fu XT, Stewart JA, Giacoletto KS, White SL, Leysath CE, Evans RJ, Shieh JJ, Karr RW (1996) Differential effects of CTLA-4 substitutions on the binding of human CD80 (B7-1) and CD86 (B7-2) J Immunol 156: 1047-1054
Muyldermans S, Atarhouch T, Saldanha J, Barbosa JA, Hamers R (1994) Sequence and structure of VH domain from naturally occurring camel heavy chain immunoglobulins lacking light chains. Protein Eng 7: 1129-1135.
Nieba L, Honegger A, Krebber C, Pluckthun A (1997) Disrupting the hydrophobic patches at the antibody variable/constant domain interface: improved in vivo folding and physical characterization of an engineered scFv fragment Protein Engineering (4):435-44
Novotny J, Ganju RK, Smiley ST, Hussey RE, Luther MA, Recny MA, Siliciano RF, Reinherz EL (1991) A soluble, single-chain T-cell receptor fragment endowed with antigen-combining properties Proc Natl Acad Sci U S A 88(19):8646-8650.
Peach RJ, Bajorath J, Brady W, Leytze G, Greene J, Naemura J, Linsley PS (1994) Complementarity determining region 1 (CDR1)- and CDR3-analogous regions in CTLA-4 and CD28 determine the binding to B7-1 J Exp Med 180: 2049-2058
Power, B.E., Ivancic, N., Harley, V.R., Webster, R.G., Kortt, A.A, Irving, R.A. and Hudson, P.J. (1992) High-level temperature-induced synthesis of an antibody VH-domain in Esclierichia coli using the PelB secretion signal gene 113:95-99.
Reisine, T. (1995) Somatostatin. Cell Molec. Neurobiol. 15: 597-614
Reubi, J. C. (1997) Regulatory peptice receptors as molecular targets for cancer diagnosis and therapy. Q. J. Nucl. Med. 41: 63-70
Schonbrunn A, Gu YZ, Brown PJ, Loose-Mitchell D (1995) Function and regulation of somatostatin receptor subtypes. Ciba Found. Symp. 190: 204-217
Smith, G. P. (1985). Filamentous fusion phage: novel expression vectors that display cloned antigens on the virion surface. Science, 228, 1315-1317
van der Merwe PA, Bodian DL, Daenke S, Linsley P, Davis SJ (1997) CD80 (B7-1) binds both CD28 and CTLA-4 with a low affinity and very fast kinetics. J Exp Med 185: 393-403
Vu KB, Ghahroudi MA, Wyns L, Muyldermans S (1997) Comparison of llama VH sequences from conventional and Heavy Chain Antibodies. Molec Immunol 34: 1121-1131
Ward ES (1991) Expression and secretion of T-cell receptor V alpha and V beta domains using Escherichia coli as a host Scand J Immunol 34(2):215-220.
Waterhouse P, Marengere LE, Mittrücker HW, Mak TW (1996) CTLA-4, a negative regulator of T-lymphocyte activation. Immunol Rev 153: 183-207
Waterhouse P, Penninger JM, Timms E, Wakeham A, Shahinian A, Lee KP, Thompson CB, Griesser H, Mak TW (1995) Lymphoproliferative disorders with early lethality in mice deficient in Ctla-4 Science 270: 985-988
Winter G, Milstein C. (1991). Man-made antibodies. Nature. 349: 293-299.
Wulfing C, Pluckthun A and in (1994) Correctly folded T-cell receptor fragments in the periplasm of Escherichia coli - Influence of folding catalysts J Mol Biol 242(5):655-69.

### SEQUENCE LISTING

Applicant: Diatech Pty Ltd
Title of Invention: V-like domain binding molecules
Prior Application Number: PP2210
   Prior Application Filing Date: 1998-03-06
Number of SEQ ID NOs: 138
Software: PatentIn Ver. 2.1
SEQ ID NO: 1
   Length: 6
   Type: PRT
   Organism: Homo sapiens
Sequence: 1
SEQ ID NO: 2
   Length: 54
   Type: DNA
   Organism: Artificial Sequence
Feature:
   Other information: Description of Artificial Sequence: Oligonucleotide PCR primer
Sequence: 2
   ttattactcg cggcccagcc ggccatggcc gcaatgcacg tggcccagcc tgct 54
SEQ ID NO: 3
   Length: 60
   Type: DNA
   Organism: Artificial Sequence
Feature:
   Other information: Description of Artificial Sequence: Oligonucleotide PCR primer
Sequence: 3
   ttattactcg cggcccagcc ggccatggcc gcaatgcacg tggcccagcc tgctgtggta 60
SEQ ID NO: 4
   Length: 45
   Type: DNA
   Organism: Artificial Sequence
Feature:
   Other information: Description of Artificial Sequence: Oligonucleotide PCR primer
Sequence: 4
   tctcacagtg cacaggcaat gcacgtggcc cagcctgctg tggta 45
SEQ ID NO: 5
   Length: 39
   Type: DNA
   Organism: Artificial Sequence
Feature:
   Other information: Description of Artificial Sequence: Oligonucleotide PCR primer
Sequence: 5
   tctcacagtg cacaggcaat gcacgtggcc cagcctgct 39
SEQ ID NO: 6
   Length: 43
   Type: DNA
   Organism: Artificial Sequence
Feature:
   Other information: Description of Artificial Sequence: Oligonucleotide PCR primer
Sequence: 6
   gcccagccgg ccgaattcgc aatgcacgtg gcccagcctg ctg 43
SEQ ID NO: 7
   Length: 60
   Type: DNA
   Organism: Artificial Sequence
Feature:
   Other information: Description of Artificial Sequence: Oligonucleotide PCR primer
Sequence: 7
   gcagctaata cgactcacta taggaacaga ccaccatgga cgtggcccag cctgctgtgg 60
SEQ ID NO: 8
   Length: 42
   Type: DNA
   Organism: Artificial Sequence
Feature:
   Other information: Description of Artificial Sequence: Oligonucleotide PCR primer
Sequence: 8
   atctgcggcc gctacataaa tctgggtacc gttgccgatg cc 42
SEQ ID NO: 9
   Length: 66
   Type: DNA
   Organism: Artificial Sequence
Feature:
   Other information: Description of Artificial Sequence: Oligonucleotide PCR primer
Sequence: 9
SEQ ID NO: 10
   Length: 51
   Type: DNA
   Organism: Artificial Sequence
Feature:
   Other information: Description of Artificial Sequence: Oligonucleotide PCR primer
Sequence: 10
   gcccttgggc cgggagatgg tctgcttcag tggcgagggc aggtctgtgt g 51
SEQ ID NO: 11
   Length: 49
   Type: DNA
   Organism: Artificial Sequence
Feature:
   Other information: Description of Artificial Sequence: Oligonucleotide PCR primer
Sequence: 11
   cgagggcagg tctgtgtggg tcacggtgca cgtgaacctc tccccggag 49
SEQ ID NO: 12
   Length: 51
   Type: DNA
   Organism: Artificial Sequence
Feature:
   Other information: Description of Artificial Sequence: Oligonucleotide PCR primer
Sequence: 12
   cgtgaacctc tccccggagt tccagtcatc ctcgcagatg ctggcctcac c 51
SEQ ID NO: 13
   Length: 84
   Type: DNA
   Organism: Artificial Sequence
Feature:
   Other information: Description of Artificial Sequence: Oligonucleotide PCR primer
Sequence: 13
SEQ ID NO: 14
   Length: 84
   Type: DNA
   Organism: Artificial Sequence
Feature:
   Other information: Description of Artificial Sequence: Oligonucleotide PCR primer
Sequence: 14
SEQ ID NO: 15
   Length: 75
   Type: DNA
   Organism: Artificial Sequence
Feature:
   Other information: Description of Artificial Sequence: Oligonucleotide PCR primer
Sequence: 15
SEQ ID NO: 16
   Length: 75
   Type: DNA
   Organism: Artificial Sequence
Feature:
   Other information: Description of Artificial Sequence: Oligonucleotide PCR primer
Sequence: 16
SEQ ID NO: 17
   Length: 21
   Type: DNA
   Organism: Artificial Sequence
Feature:
   Other information: Description of Artificial Sequence: Oligonucleotide PCR primer
Sequence: 17
   gtaggttgcc gcacagactt c 21
SEQ ID NO: 18
   Length: 66
   Type: DNA
   Organism: Artificial Sequence
Feature:
   Other information: Description of Artificial Sequence: Oligonucleotide PCR primer
Sequence: 18
SEQ ID NO: 19
   Length: 78
   Type: DNA
   Organism: Artificial Sequence
Feature:
   Other information: Description of Artificial Sequence: Oligonucleotide PCR primer
Sequence: 19
SEQ ID NO: 20
   Length: 60
   Type: DNA
   Organism: Artificial Sequence
Feature:
   Other information: Description of Artificial Sequence: Oligonucleotide PCR primer
Sequence: 20
   tgtgcggcag ccatcaacat gggcggtggc atcaccttcc tagatgattc catctgcacg 60
SEQ ID NO: 21
   Length: 60
   Type: DNA
   Organism: Artificial Sequence
Feature:
   Other information: Description of Artificial Sequence: Oligonucleotide PCR primer
Sequence: 21
   atctaggaag gtgatgccac cgcccatgtt gatggctgcc gcacagactt cagtcacctg 60
SEQ ID NO: 22
   Length: 69
   Type: DNA
   Organism: Artificial Sequence
Feature:
   Other information: Description of Artificial Sequence: Oligonucleotide PCR primer
Sequence: 22
SEQ ID NO: 23
   Length: 72
   Type: DNA
   Organism: Artificial Sequence
Feature:
   Other information: Description of Artificial Sequence: Oligonucleotide PCR primer
Sequence: 23
SEQ ID NO: 24
   Length: 78
   Type: DNA
   Organism: Artificial Sequence
Feature:
   Other information: Description of Artificial Sequence: Oligonucleotide PCR primer
Sequence: 24
SEQ ID NO: 25
   Length: 51
   Type: DNA
   Organism: Artificial Sequence
Feature:
   Other information: Description of Artificial Sequence: Oligonucleotide PCR primer
Sequence: 25
   gccatctccg gatccggagg ctcgacctac ctagatgatt ccatctgcac g 51
SEQ ID NO: 26
   Length: 54
   Type: DNA
   Organism: Artificial Sequence
Feature:
   Other information: Description of Artificial Sequence: Oligonucleotide PCR primer
Sequence: 26
   gtaggtcgag cctccggatc cggagatggc tgccgcacag acttcagtca cctg 54
SEQ ID NO: 27
   Length: 69
   Type: DNA
   Organism: Artificial Sequence
Feature:
   Other information: Description of Artificial Sequence: Oligonucleotide PCR primer
Sequence: 27
SEQ ID NO: 28
   Length: 51
   Type: DNA
   Organism: Artificial Sequence
Feature:
   Other information: Description of Artificial Sequence: Oligonucleotide PCR primer
Sequence: 28
   aatctgggta ccgttgccga tgcccacgtc catgtagtag tctccctcct c 51
SEQ ID NO: 29
   Length: 66
   Type: DNA
   Organism: Artificial Sequence
Feature:
   Other information: Description of Artificial Sequence: Oligonucleotide PCR primer
Sequence: 29
SEQ ID NO: 30
   Length: 68
   Type: DNA
   Organism: Artificial Sequence
Feature:
   Other information: Description of Artificial Sequence: Oligonucleotide PCR primer
Sequence: 30
SEQ ID NO: 31
   Length: 66
   Type: DNA
   Organism: Artificial Sequence
Feature:
   Other information: Description of Artificial Sequence: Oligonucleotide PCR primer
Sequence: 31
SEQ ID NO: 32
   Length: 73
   Type: DNA
   Organism: Artificial Sequence
Feature:
   Other information: Description of Artificial Sequence: Oligonucleotide PCR primer
Sequence: 32
SEQ ID NO: 33
   Length: 82
   Type: DNA
   Organism: Artificial Sequence
Feature:
   Other information: Description of Artificial Sequence: Oligonucleotide PCR Primer
Sequence: 33
SEQ ID NO: 34
   Length: 82
   Type: DNA
   Organism: Artificial Sequence
Feature:
   Other information: Description of Artificial Sequence: Oligonucleotide PCR Primer
Sequence: 34
SEQ ID NO: 35
   Length: 70
   Type: DNA
   Organism: Artificial Sequence
Feature:
   Other information: Description of Artificial Sequence: Oligonucleotide PCR primer
Sequence: 35
SEQ ID NO: 36
   Length: 70
   Type: DNA
   Organism: Artificial Sequence
Feature:
   Other information: Description of Artificial Sequence: Oligonucleotide PCR primer
Sequence: 36
SEQ ID NO: 37
   Length: 68
   Type: DNA
   Organism: Artificial Sequence
Feature:
   Other information: Description of Artificial Sequence: Oligonucleotide PCR primer
Sequence: 37
SEQ ID NO: 38
   Length: 30
   Type: DNA
   Organism: Artificial Sequence
Feature:
   Other information: Description of Artificial Sequence: Oligonucleotide PCR primer
Sequence: 38
   gtaggttgcc gcacagactt cagtcacctg 30
SEQ ID NO: 39
   Length: 68
   Type: DNA
   Organism: Artificial Sequence
Feature:
   Other information: Description of Artificial Sequence: Oligonucleotide PCR primer
Sequence: 39
SEQ ID NO: 40
   Length: 30
   Type: DNA
   Organism: Artificial Sequence
Feature:
   Other information: Description of Artificial Sequence: Oligonucleotide PCR primer
Sequence: 40
   gtagcatgcc gcacagactt cagtcacctg 30
SEQ ID NO: 41
   Length: 69
   Type: DNA
   Organism: Artificial Sequence
Feature:
   Other information: Description of Artificial Sequence: Oligonucleotide PCR primer
Sequence: 41
SEQ ID NO: 42
   Length: 67
   Type: DNA
   Organism: Artificial Sequence
Feature:
   Other information: Description of Artificial Sequence: Oligonucleotide PCR primer
Sequence: 42
SEQ ID NO: 43
   Length: 78
   Type: DNA
   Organism: Artificial Sequence
Feature:
   Other information: Description of Artificial Sequence: Oligonucleotide PCR primer
Sequence: 43
SEQ ID NO: 44
   Length: 93
   Type: DNA
   Organism: Artificial Sequence
Feature:
   Other information: Description of Artificial Sequence: Oligonucleotide PCR primer
Sequence: 44
SEQ ID NO: 45
   Length: 81
   Type: DNA
   Organism: Artificial Sequence
Feature:
   Other information: Description of Artificial Sequence: Oligonucleotide PCR primer
Sequence: 45
SEQ ID NO: 46
   Length: 87
   Type: DNA
   Organism: Artificial Sequence
Feature:
   Other information: Description of Artificial Sequence: Oligonucleotide PCR primer
Sequence: 46
SEQ ID NO: 47
   Length: 99
   Type: DNA
   Organism: Artificial Sequence
Feature:
   Other information: Description of Artificial Sequence: Oligonucleotide PCR primer
Sequence: 47
SEQ ID NO: 48
   Length: 87
   Type: DNA
   Organism: Artificial Sequence
Feature:
   Other information: Description of Artificial Sequence: Oligonucleotide PCR primer
Sequence: 48
SEQ ID NO: 49
   Length: 70
   Type: DNA
   Organism: Artificial Sequence
Feature:
   Other information: Description of Artificial Sequence: Oligonucleotide PCR primer
Sequence: 49
SEQ ID NO: 50
   Length: 77
   Type: DNA
   Organism: Artificial Sequence
Feature:
   Other information: Description of Artificial Sequence: Oligonucleotide PCR primer
Sequence: 50
SEQ ID NO: 51
   Length: 54
   Type: DNA
   Organism: Artificial Sequence
Feature:
   Other information: Description of Artificial Sequence: Oligonucleotide PCR primer
Sequence: 51
   gtgcgtgtga ccgtgctgcg tcaggcggat agccaggtga ccgaagtttg cgcg 54
SEQ ID NO: 52
   Length: 75
   Type: DNA
   Organism: Artificial Sequence
Feature:
   Other information: Description of Artificial Sequence: Oligonucleotide PCR primer
Sequence: 52
SEQ ID NO: 53
   Length: 66
   Type: DNA
   Organism: Artificial Sequence
Feature:
   Other information: Description of Artificial Sequence: Oligonucleotide PCR primer
Sequence: 53
SEQ ID NO: 54
   Length: 57
   Type: DNA
   Organism: Artificial Sequence
Feature:
   Other information: Description of Artificial Sequence: Oligonucleotide PCR primer
Sequence: 54
   cactttgcag atgtacagac cggtatccat ggcacgcaga ccctggatgg tcaggtt 57
SEQ ID NO: 55
   Length: 66
   Type: DNA
   Organism: Artificial Sequence
Feature:
   Other information: Description of Artificial Sequence: Oligonucleotide PCR primer
Sequence: 55
SEQ ID NO: 56
   Length: 69
   Type: DNA
   Organism: Artificial Sequence
Feature:
   Other information: Description of Artificial Sequence: Oligonucleotide PCR primer
Sequence: 56
SEQ ID NO: 57
   Length: 672
   Type: DNA
   Organism: Homo sapiens
Sequence: 57
SEQ ID NO: 58
   Length: 115
   Type: PRT
   Organism: Homo sapiens
Sequence: 58
SEQ ID NO: 59
   Length: 7
   Type: PRT
   Organism: Homo sapiens
Sequence: 59
SEQ ID NO: 60
   Length: 9
   Type: PRT
   Organism: Homo sapiens
Sequence: 60
SEQ ID NO: 61
   Length: 9
   Type: PRT
   Organism: Homo sapiens
Sequence: 61
SEQ ID NO: 62
   Length: 14
   Type: PRT
   Organism: Homo sapiens
Sequence: 62
SEQ ID NO: 63
   Length: 9
   Type: PRT
   Organism: Artificial Sequence
Feature:
   Other information: Description of Artificial Sequence:Haemagglutinin tag
Sequence: 63
SEQ ID NO: 64
   Length: 11
   Type: PRT
   Organism: Artificial Sequence
Feature:
   Other information: Description of Artificial Sequence: Sequence from anti-lysozyme antibody
Sequence: 64
SEQ ID NO: 65
   Length: 10
   Type: PRT
   Organism: Artificial Sequence
Feature:
   Other information: Description of Artificial Sequence: Sequence from anti-lysozyme antibody
Sequence: 65
SEQ ID NO: 66
   Length: 24
   Type: PRT
   Organism: Artificial Sequence
Feature:
   Other information: Description of Artificial Sequence: Sequence from anti-lysozyme antibody
Sequence: 66
SEQ ID NO: 67
   Length: 11
   Type: PRT
   Organism: Homo sapiens
Sequence: 67
SEQ ID NO: 68
   Length: 10
   Type: PRT
   Organism: Homo sapiens
Sequence: 68
SEQ ID NO: 69
   Length: 15
   Type: PRT
   Organism: Homo sapiens
Sequence: 69
SEQ ID NO: 70
   Length: 21
   Type: PRT
   Organism: Artificial Sequence
Feature:
   Other information: Description of Artificial Sequence: Flag-tag
Sequence: 70
SEQ ID NO: 71
   Length: 14
   Type: PRT
   Organism: Homo sapiens
Sequence: 71
SEQ ID NO: 72
   Length: 18
   Type: PRT
   Organism: Artificial Sequence
Feature:
   Other information: Description of Artificial Sequence:Fusion protein
Sequence: 72
SEQ ID NO: 73
   Length: 24
   Type: PRT
   Organism: Artificial Sequence
Feature:
   Other information: Description of Artificial Sequence:Fusion protein
Sequence: 73
SEQ ID NO: 74
   Length: 18
   Type: PRT
   Organism: Artificial Sequence
Feature:
   Other information: Description of Artificial Sequence:Fusion protein
Sequence: 74
SEQ ID NO: 75
   Length: 15
   Type: PRT
   Organism: Artificial Sequence
Feature:
   Other information: Description of Artificial Sequence:Fusion protein containing random sequence
Sequence: 75
SEQ ID NO: 76
   Length: 18
   Type: PRT
   Organism: Artificial Sequence
Feature:
   Other information: Description of Artificial Sequence:Fusion protein containing random sequence
Sequence: 76
SEQ ID NO: 77
   Length: 21
   Type: PRT
   Organism: Artificial Sequence
Feature:
   Other information: Description of Artificial Sequence:Fusion protein containing random sequence
Sequence: 77
SEQ ID NO: 78
   Length: 14
   Type: PRT
   Organism: Artificial Sequence
Feature:
   Other information: Description of Artificial Sequence:Fusion protein containing random sequence
Sequence: 78
SEQ ID NO: 79
   Length: 14
   Type: PRT
   Organism: Artificial Sequence
Feature:
   Other information: Description of Artificial Sequence:Fusion protein containing random sequence
Sequence: 79
SEQ ID NO: 80
   Length: 18
   Type: PRT
   Organism: Artificial Sequence
Feature:
   Other information: Description of Artificial Sequence:Fusion protein containing random sequence
Sequence: 80
SEQ ID NO: 81
   Length: 18
   Type: PRT
   Organism: Artificial Sequence
Feature:
   Other information: Description of Artificial Sequence:Fusion protein containing random sequence
Sequence: 81
SEQ ID NO: 82
   Length: 24
   Type: PRT
   Organism: Artificial Sequence
Feature:
   Other information: Description of Artificial Sequence:Fusion protein containing random sequence
Sequence: 82
SEQ ID NO: 83
   Length: 25
   Type: PRT
   Organism: Homo sapiens
Sequence: 83
SEQ ID NO: 84
   Length: 25
   Type: PRT
   Organism: Artificial Sequence
Feature:
   Other information: Description of Artificial Sequence: Fusion protein
Sequence: 84
SEQ ID NO: 85
   Length: 25
   Type: PRT
   Organism: Artificial Sequence
Feature:
   Other information: Description of Artificial Sequence: Fusion protein
Sequence: 85
SEQ ID NO: 86
   Length: 25
   Type: PRT
   Organism: Artificial Sequence
Feature:
   Other information: Description of Artificial Sequence: Fusion protein
Sequence: 86
SEQ ID NO: 87
   Length: 25
   Type: PRT
   Organism: Artificial Sequence
Feature:
   Other information: Description of Artificial Sequence: Fusion protein containing random sequence
Sequence: 87
SEQ ID NO: 88
   Length: 25
   Type: PRT
   Organism: Artificial Sequence
Feature:
   Other information: Description of Artificial Sequence: Fusion protein containing random sequence
Sequence: 88
SEQ ID NO: 89
   Length: 13
   Type: PRT
   Organism: Homo sapiens
Sequence: 89
SEQ ID NO: 90
   Length: 27
   Type: PRT
   Organism: Artificial Sequence
Feature:
   Other information: Description of Artificial Sequence: Fusion protein
Sequence: 90
SEQ ID NO: 91
   Length: 18
   Type: PRT
   Organism: Artificial Sequence
Feature:
   Other information: Description of Artificial Sequence: Fusion protein
Sequence: 91
SEQ ID NO: 92
   Length: 18
   Type: PRT
   Organism: Artificial Sequence
Feature:
   Other information: Description of Artificial Sequence: Fusion protein
Sequence: 92
SEQ ID NO: 93
   Length: 14
   Type: PRT
   Organism: Artificial Sequence
Feature:
   Other information: Description of Artificial Sequence: Fusion protein containing random sequence
Sequence: 93
SEQ ID NO: 94
   Length: 18
   Type: PRT
   Organism: Artificial Sequence
Feature:
   Other information: Description of Artificial Sequence: Fusion protein containing random sequence
Sequence: 94
SEQ ID NO: 95
   Length: 14
   Type: PRT
   Organism: Artificial Sequence
Feature:
   Other information: Description of Artificial Sequence: Fusion protein containing random sequence
Sequence: 95
SEQ ID NO: 96
   Length: 15
   Type: PRT
   Organism: Artificial Sequence
Feature:
   Other information: Description of Artificial Sequence: Fusion protein containing random sequence
Sequence: 96
SEQ ID NO: 97
   Length: 17
   Type: PRT
   Organism: Artificial Sequence
Feature:
   Other information: Description of Artificial Sequence: Fusion protein containing random sequence
Sequence: 97
SEQ ID NO: 98
   Length: 21
   Type: PRT
   Organism: Artificial Sequence
Feature:
   Other information: Description of Artificial Sequence: Fusion protein containing random sequence
Sequence: 98
SEQ ID NO: 99
   Length: 13
   Type: PRT
   Organism: Artificial Sequence
Feature:
   Other information: Description of Artificial Sequence: Fusion protein containing random sequence
Sequence: 99
SEQ ID NO: 100
   Length: 18
   Type: PRT
   Organism: Artificial Sequence
Feature:
   Other information: Description of Artificial Sequence: Fusion protein containing random sequence
Sequence: 100
SEQ ID NO: 101
   Length: 18
   Type: PRT
   Organism: Artificial Sequence
Feature:
   Other information: Description of Artificial Sequence: Fusion protein containing random sequence
Sequence: 101
SEQ ID NO: 102
   Length: 10
   Type: PRT
   Organism: Artificial Sequence
Feature:
   Other information: Description of Artificial Sequence: CDR1 and CDR3 inserts possessing randomly generated sequence
Sequence: 102
SEQ ID NO: 103
   Length: 8
   Type: PRT
   Organism: Artificial Sequence
Feature:
   Other information: Description of Artificial Sequence: CDR1 and CDR3 inserts possessing randomly generated sequence
Sequence: 103
SEQ ID NO: 104
   Length: 10
   Type: PRT
   Organism: Artificial Sequence
Feature:
   Other information: Description of Artificial Sequence: CDR1 and CDR3 inserts possessing randomly generated sequence
Sequence: 104
SEQ ID NO: 105
   Length: 7
   Type: PRT
   Organism: Artificial Sequence
Feature:
   Other information: Description of Artificial Sequence: CDR1 and CDR3 inserts possessing randomly generated sequence
Sequence: 105
SEQ ID NO: 106
   Length: 8
   Type: PRT
   Organism: Artificial Sequence
Feature:
   Name/Key: MOD_RES
   Location: (5)
   Other information: Stop codon but Glu when expressed in Tg-1 or JM109 strains of E.coli
Feature:
   Other information: Description of Artificial Sequence: CDR1 and CDR3 inserts possessing randomly generated sequence
Sequence: 106
SEQ ID NO: 107
   Length: 10
   Type: PRT
   Organism: Artificial Sequence
Feature:
   Other information: Description of Artificial Sequence: CDR1 and CDR3 inserts possessing randomly generated sequence
Sequence: 107
SEQ ID NO: 108
   Length: 8
   Type: PRT
   Organism: Artificial Sequence
Feature:
   Name/Key: MOD_RES
   Location: (4)
   Other information: Stop codon but Glu when expressed in Tg-1 of JM109 E.coli strains
Feature:
   Other information: Description of Artificial Sequence: CDR1 and CDR3 inserts possessing randomly generated sequence
Sequence: 108
SEQ ID NO: 109
   Length: 8
   Type: PRT
   Organism: Artificial Sequence
Feature:
   Other information: Description of Artificial Sequence: CDR1 and CDR3 inserts possessing randomly generated sequence
Sequence: 109
SEQ ID NO: 110
   Length: 7
   Type: PRT
   Organism: Artificial Sequence
Feature:
   Name/Key: MOD_RES
   Location: (7)
   Other information: Stop codon but Glu when expressed in Tg-1 or JM109 strains of E. coli
Feature:
   Other information: Description of Artificial Sequence: CDR1 and CDR3 inserts possessing randomly generated sequence
Sequence: 110
SEQ ID NO: 111
   Length: 7
   Type: PRT
   Organism: Artificial Sequence
Feature:
   Other information: Description of Artificial Sequence: CDR1 and CDR3 inserts possessing randomly generated sequence
Sequence: 111
SEQ ID NO: 112
   Length: 7
   Type: PRT
   Organism: Artificial Sequence
Feature:
   Other information: Description of Artificial Sequence: CDR1 and CDR3 inserts possessing randomly generated sequence
Sequence: 112
SEQ ID NO: 113
   Length: 11
   Type: PRT
   Organism: Artificial Sequence
Feature:
   Name/Key: MOD_RES
   Location: (8)
   Other information: Stop codon but Glu when expressed in Tg-1 or JM109 strains of E. coli
Feature:
   Other information: Description of Artificial Sequence: CDR1 and CDR3 inserts possessing randomly generated sequence
Sequence: 113
SEQ ID NO: 114
   Length: 11
   Type: PRT
   Organism: Artificial Sequence
Feature:
   Other information: Description of Artificial Sequence: CDR1 and CDR3 inserts possessing randomly generated sequence
Sequence: 114
SEQ ID NO: 115
   Length: 10
   Type: PRT
   Organism: Artificial Sequence
Feature:
   Other information: Description of Artificial Sequence: CDR1 and CDR3 inserts possessing randomly generated sequence
Sequence: 115
SEQ ID NO: 116
   Length: 7
   Type: PRT
   Organism: Artificial Sequence
Feature:
   Other information: Description of Artificial Sequence: CDR1 and CDR3 inserts possessing randomly generated sequence
Sequence: 116
SEQ ID NO: 117
   Length: 13
   Type: PRT
   Organism: Artificial Sequence
Feature:
   Other information: Description of Artificial Sequence: CDR1 and CDR3 inserts possessing randomly generated sequence
Sequence: 117
SEQ ID NO: 118
   Length: 7
   Type: PRT
   Organism: Artificial Sequence
Feature:
   Name/Key: MOD_RES
   Location: (4)
   Other information: Stop codon but Glu when expressed in Tg-1 or JM109 strains of E. coli
Feature:
   Other information: Description of Artificial Sequence: CDR1 and CDR3 inserts possessing randomly generated sequence
Sequence: 118
SEQ ID NO: 119
   Length: 13
   Type: PRT
   Organism: Artificial Sequence
Feature:
   Other information: Description of Artificial Sequence: CDR1 and CDR3 inserts possessing randomly generated sequence
Sequence: 119
SEQ ID NO: 120
   Length: 13
   Type: PRT
   Organism: Artificial Sequence
Feature:
   Other information: Description of Artificial Sequence: CDR1 and CDR3 inserts possessing randomly generated sequence
Sequence: 120
SEQ ID NO: 121
   Length: 7
   Type: PRT
   organism: Artificial Sequence
Feature:
   Other information: Description of Artificial Sequence: CDR1 and CDR3 inserts possessing randomly generated sequence
Sequence: 121
SEQ ID NO: 122
   Length: 13
   Type: PRT
   Organism: Artificial Sequence
Feature:
   Other information: Description of Artificial Sequence: CDR1 and CDR3 inserts possessing randomly generated sequence
Sequence: 122
SEQ ID NO: 123
   Length: 13
   Type: PRT
   Organism: Artificial Sequence
Feature:
   Other information: Description of Artificial Sequence: CDR1 and CDR3 inserts possessing randomly generated sequence
Sequence: 123
SEQ ID NO: 124
   Length: 10
   Type: PRT
   Organism: Artificial Sequence
Feature:
   Other information: Description of Artificial Sequence: CDR1 and CDR3 inserts possessing randomly generated sequence
Sequence: 124
SEQ ID NO: 125
   Length: 7
   Type: PRT
   Organism: Artificial Sequence
Feature:
   Other information: Description of Artificial Sequence: CDR1 and CDR3 inserts possessing randomly generated sequence
Sequence: 125
SEQ ID NO: 126
   Length: 14
   Type: PRT
   Organism: Artificial Sequence
Feature:
   Other information: Description of Artificial Sequence: CDR1 and CDR3 inserts possessing randomly generated sequence
Sequence: 126
SEQ ID NO: 127
   Length: 11
   Type: PRT
   Organism: Artificial Sequence
Feature:
   Other information: Description of Artificial Sequence: CDR1 and CDR3 inserts possessing randomly generated sequence
Sequence: 127
SEQ ID NO: 128
   Length: 15
   Type: PRT
   Organism: Artificial Sequence
Feature:
   Name/Key: MOD_RES
   Location: (7)
   Other information: Stop codon but Glu when expressed in Tg-1 of JM019 strains of E. coli
Feature:
   Other information: Description of Artificial Sequence: CDR1 and CDR3 inserts possessing randomly generated sequence
Sequence: 128
SEQ ID NO: 129
   Length: 7
   Type: PRT
   Organism: Artificial Sequence
Feature:
   Name/Key: MOD_RES
   Location: (6)
   Other information: Stop codon but Glu when expressed in Tg-1 or JM109 strains of E. coli
Feature:
   Other information: Description of Artificial Sequence: CDR1 and CDR3 inserts possessing randomly generated sequence
Sequence: 129
SEQ ID NO: 130
   Length: 8
   Type: PRT
   Organism: Artificial Sequence
Feature:
   Other information: Description of Artificial Sequence: CDR1 and CDR3 inserts possessing randomly generated sequence
Sequence: 130
SEQ ID NO: 131
   Length: 7
   Type: PRT
   Organism: Artificial Sequence
Feature:
   Other information: Description of Artificial Sequence: CDR1 and CDR3 inserts possessing randomly generated sequence
Sequence: 131
SEQ ID NO: 132
   Length: 15
   Type: PRT
   Organism: Artificial Sequence
Feature:
   Other information: Description of Artificial Sequence: CDR1 and CDR3 inserts possessing randomly generated sequence
Sequence: 132
SEQ ID NO: 133
   Length: 8
   Type: PRT
   Organism: Artificial Sequence
Feature:
   Other information: Description of Artificial Sequence: CDR1 and CDR3 inserts possessing randomly generated sequence
Sequence: 133
SEQ ID NO: 134
   Length: 10
   Type: PRT
   Organism: Artificial Sequence
Feature:
   Other information: Description of Artificial Sequence: CDR1 and CDR3 inserts possessing randomly generated sequence
Sequence: 134
SEQ ID NO: 135
   Length: 8
   Type: PRT
   Organism: Artificial Sequence
Feature:
   Other information: Description of Artificial Sequence: CDR1 and CDR3 inserts possessing randomly generated sequence
Sequence: 135
SEQ ID NO: 136
   Length: 12
   Type: PRT
   Organism: Artificial Sequence
Feature:
   Other information: Description of Artificial Sequence: CDR1 and CDR3 inserts possessing randomly generated sequence
Sequence: 136
SEQ ID NO: 137
   Length: 7
   Type: PRT
   Organism: Artificial Sequence
Feature:
   Other information: Description of Artificial Sequence: CDR1 and CDR3 inserts possessing randomly generated sequence
Sequence: 137
SEQ ID NO: 138
   Length: 15
   Type: PRT
   Organism: Artificial Sequence
Feature:
   Other information: Description of Artificial Sequence: CDR1 and CDR3 inserts possessing randomly generated sequence
Sequence: 138

## Claims

1. A binding moiety comprising at least one monomeric non-antibody ligand V-like domain (VLD), the at least one monomeric V-like domain being **characterised in that** at least one CDR loop structure or part thereof is modified or replaced such that
(i) the size of the CDR loop structure is increased when compared with the corresponding CDR loop structure in the unmodified VLD; and/or
(ii) the modification or replacement results in the formation of a disulphide bond within or between one or more of the CDR loop structures.

2. A binding moiety according to claim 1 in which the size of the CDR loop structure is increased by one amino acid residue.

3. A binding moiety according to claim 1 in which the size of the CDR loop structure is increased by at least two amino acid structures.

4. A binding moiety according to claim 1 in which the size of the CDR loop structure is increased by at least six amino acid residues.

5. A binding moiety according to claim 1 in which the size of the CDR loop structure is increased by at least nine amino acid residues.

6. A binding according to any one of claims 1 to 5 in which the solubility of the modified VLD is improved when compared with the unmodified VLD.

7. A binding moiety according to any one of claims 1 to 6 in which the binding affinity of the modified VLD is altered when compared with the unmodified VLD.

8. A binding moiety according to claim 7 in which the affinity of the modified VLD to at least one natural ligand of the unmodified VLD is reduced.

9. A binding moiety according to any one of claims 1 to 8 in which the binding specificity of the modified VLD is different to that of the unmodified VLD.

10. A binding moiety according to any one of claims 1 to 9 in which the non-antibody ligand is a T-cell surface protein.

11. A binding moiety according to claim 10 in which the non-antibody is CTLA-4, CD28 or ICOS.

12. A binding moiety according to claim 11 in which the non-antibody ligand is CTLA-4.

13. A binding moiety according to any one of claims 1 to 12 in which one or more of the CDR loop structures is replaced with a binding determinant derived from a non-antibody polypeptide.

14. A binding moiety according to claim 13 in which the binding determinant is derived from somatostatin or haemagglutinin.

15. A binding moiety according to any one of claims 1 to 12 in which one or more of the CDR loop structures is replaced with one or more CDR loop structures derived from an antibody or antibodies.

16. A binding moiety according to claim 15 in which the antibody or antibodies are derived from a rat, mouse, human, camel, llama or shark.

17. A binding moiety according to claim 15 or claim 16 in which the antibody or antibodies are selected from the camel antibody cAB-Lys3 and the human anti-melanoma antibody V86.

18. A binding moiety according to any one of claims 1 to 17 linked to a diagnostic reagent.

19. A binding moiety according to claim 18 in which the diagnostic reagent is selected from the group consisting of streptavidin, biotin, a radioisotope, dye marker or other imaging reagent.

20. A reagent comprising two or more binding moieties according to any one of the preceding claims coupled together either chemically or genetically.

21. A binding moiety or reagent according to any one of claims 1 to 20 immobilised on a solid support or coupled to a biosensor surface.

22. A polynucleotide encoding a binding moiety or reagent as claimed in any one of claims 1 to 20.

23. A vector comprising a polynucleotide according to claim 22.

24. A host cell comprising a vector as claimed in claim 23.

25. A host cell according to claim 24 in which the cell is a bacterial cell.

26. A method of producing a binding moiety which comprises culturing a host cell as claimed in claim 24 or claim 25 under conditions enabling expression of the binding moiety and optionally recovering the binding moiety.

27. A method according to claim 26 in which the binding moiety is unglycosylated.

28. A pharmaceutical composition comprising a binding moiety as claimed in any one of claims 1 to 20 and a pharmaceutically acceptable carrier or diluent.

29. A binding moiety or reagent as claimed in any one of claims 1 to 20 for use in treating a pathological condition in a subject.

30. A method of selecting a binding moiety with an affinity for a target molecule which comprises screening a library of polynucleotides for expression of a binding moiety with an affinity for the target molecule, the polynucleotides encoding one or more non-antibody ligand VLDs, wherein the polynucleotides have been subjected to mutagenesis which results in a modification or replacement in at least one CDR loop structure in at least one VLD such that
(i) the size of the CDR loop structure is increased when compared with the corresponding CDR loop structure in the unmodified VLD; and/or
(ii) the modification or replacement results in the formation of a disulphide bond within or between one or more of the CDR loop structures.

31. A method according to claim 30 in which the solubility of the isolated modified VLD is improved when compared with the isolated unmodified VLD.

32. A method according to claim 31 or claim 32 in which the screening process involves displaying the modified V-like domains as gene III protein fusions on the surface of bacteriophage particles.

33. A method according to claim 31 or claim 32 in which the screening process involves displaying the modified V-like domains in a ribosomal display selection system.

## Patentansprüche

1. Bindungsgruppe, umfassend wenigstens eine monomere nicht-antikörper V-ähnliche Ligandendomäne (VLD), wobei die zumindest eine monomere V-ähnliche Domäne **dadurch gekennzeichnet ist, dass** zumindest eine CDR-Loopstruktur oder ein Teil hiervon modifiziert oder ersetzt ist, so dass
(i) die Größe der CDR-Loopstruktur vergrößert ist, verglichen mit der entsprechenden CDR-Loopstruktur in der nicht-modifizierten VLD; und/oder
(ii) die Modifikation oder das Ersetzen in der Bildung einer Disulfidbindung in oder zwischen einer oder mehreren der CDR-Loopstrukturen resultiert.

2. Bindungsgruppe nach Anspruch 1, in welcher die Größe der CDR-Loopstruktur durch einen Aminosäurerest vergrößert ist.

3. Bindungsgruppe nach Anspruch 1, in welcher die Größe der CDR-Loopstruktur durch mindestens zwei Aminosäurestrukturen vergrößert ist.

4. Bindungsgruppe nach Anspruch 1, in welcher die Größe der CDR-Loopstruktur durch mindestens sechs Aminosäurereste vergrößert ist.

5. Bindungsgruppe nach Anspruch 1, in welcher die Größe der CDR-Loopstruktur durch mindestens neun Aminosäurereste vergrößert ist.

6. Bindungsgruppe nach einem Ansprüche 1 bis 5, in welcher die Löslichkeit der modifizierten VLD im Vergleich mit der nicht-modifizierten VLD verbessert ist.

7. Bindungsgruppe nach einem der Ansprüche 1 bis 6, in welcher die Bindungsaffinität der modifizierten VLD im Vergleich mit der nicht-modifizierten VLD verändert ist.

8. Bindungsgruppe nach Anspruch 7, in welcher die Affinität der modifizierten VLD an zumindest einen natürlichen Liganden der nicht-modifizierten VLD verringert ist.

9. Bindungsgruppe nach einem der Ansprüche 1 bis 8, in welcher die Bindungsspezifität der modifizierten VLD zu der der nicht-modifizierten VLD unterschiedlich ist.

10. Bindungsgruppe nach einem der Ansprüche 1 bis 9, in welcher der Nicht-Antikörper-Ligand ein T-Zell-Oberflächenprotein ist.

11. Bindungsgruppe nach Anspruch 10, in welcher der Nicht-Antkörper CTLA-4, CD28 oder ICOS ist.

12. Bindungsgruppe nach Anspruch 11, in welcher der Nicht-Antikörper-Ligand CTLA-4 ist.

13. Bindungsgruppe nach einem der Ansprüche 1 bis 12, in welcher eine oder mehrere der CDR-Loopstrukturen durch eine Bindungsdeterminante ersetzt ist, die von einem Nicht-Antikörper-Polypeptid stammt.

14. Bindungsgruppe nach Anspruch 13, in welcher die Bindungsdeterminante von Somatostatin oder Hämagglutinin stammt.

15. Bindungsgruppe nach einem der Ansprüche 1 bis 12, in welcher eine oder mehrere der CDR-Loopstrukturen durch eine oder mehrere CDR-Loopstrukturen ersetzt ist, die von einem Antikörper oder Antikörpern stammen.

16. Bindungsgruppe nach Anspruch 15, in welcher der Antikörper oder die Antikörper von einer Ratte, Maus, Menschen, Kamel, Lama oder Haifisch stammen.

17. Bindungsgruppe nach Anspruch 15 oder Anspruch 16, in welcher der Antikörper oder die Antikörper ausgewählt sind aus dem Kamelantikörper cAB-Lys3 und dem Human-Anti-Melanom-Antikörper V86.

18. Bindungsgruppe nach einem der Ansprüche 1 bis 17, verbunden mit einem Diagnostikreagenz.

19. Bindungsgruppe nach Anspruch 18, in welcher das Diagnostikreagenz ausgewählt ist aus der Gruppe, bestehend aus Streptavidin, Biotin, einem Radioisotop, einem Farbstoffmarker oder einem anderen bildgebenden Reagenz.

20. Reagenz, umfassend zwei oder mehr Bindungsgruppen nach irgendeinem der vorhergehenden Ansprüche, verbunden entweder chemisch oder genetisch.

21. Bindungsgruppe oder Reagenz nach irgendeinem der Ansprüche 1 bis 20, die bzw. das an einem Festträger immobilisiert ist oder an eine Biosensoroberfläche gekoppelt vorliegt.

22. Polynukleotid, welches für eine Bindungsgruppe oder ein Reagenz nach irgendeinem der Ansprüche 1 bis 20 kodiert.

23. Vektor, der ein Polynukleotid nach Anspruch 22 umfasst.

24. Wirtszelle, die einen Vektor nach Anspruch 23 umfasst.

25. Wirtszelle nach Anspruch 24, in welcher die Zelle eine Bakterienzelle ist.

26. Verfahren zur Herstellung einer Bindungsgruppe, umfassend das Kultivieren einer Wirtszelle nach Anspruch 24 oder Anspruch 25 unter solchen Bedingungen, die eine Expression der Bindungsgruppe ermöglichen und, wahlweise, ein Wiedergewinnen der Bindungsgruppe ermöglichen.

27. Verfahren nach Anspruch 26, in welcher die Bindungsgruppe nicht glykosyliert ist.

28. Pharmazeutische Zusammensetzung, umfassend eine Bindungsgruppe nach irgendeinem der Ansprüche 1 bis 20 und einen pharmazeutisch verträglichen Träger oder ein pharmazeutisch verträgliches Verdünnungsmittel.

29. Bindungsgruppe oder Reagenz nach irgendeinem der Ansprüche 1 bis 20 zur Verwendung bei der Behandlung eines pathologischen Zustandes in einem Subjekt.

30. Verfahren zum Auswählen einer Bindungsgruppe mit einer Affinität an ein Targetmolekül, umfassend das Screening einer Bibliothek an Polynukleotiden zur Expression einer Bindungsgruppe mit einer Affinität an ein Targetmolekül, wobei die Polynukleotide ein oder mehrere Nicht-Antikörper-VLDs-Liganden kodieren, wobei die Polynukleotide einer Mutagenese unterzogen wurden, die zu einem Modifikation oder zu einem Ersatz in zumindest einer CDR-Loopstruktur in zumindest einem VLD führen, so dass
(i) die Größe der CDR-Loopstruktur vergrößert ist, verglichen mit der entsprechenden CDR-Loopstruktur in der nicht-modifizierten VLD; und/oder
(ii) die Modifikation oder das Ersetzen in der Bildung einer Disulfidbindung in oder zwischen einer oder mehreren der CDR-Loopstrukturen resultiert.

31. Verfahren nach Anspruch 30, in welchem die Löslichkeit der isolierten modifizierten VLD verglichen mit der isolierten nicht-modifizierten VLD verbessert ist.

32. Verfahren nach Anspruch 31 oder Anspruch 32, in welchem der Screeningsprozess das Präsentieren der modifizierten V-ähnlichen Domänen als Gen III-Proteinfusionen auf der Oberfläche von Bakteriophagenteilchen umfasst.

33. Verfahren nach Anspruch 31 oder Anspruch 32, in welchem der Screeningsprozess das Präsentieren der modifizierten V-ähnlichen Domänen in einem Ribosomendisplay-Selektionssystem umfasst.

## Revendications

1. Fraction de fixation comprenant au moins un domaine de type V ligand non anticorps monomère (VLD), le domaine de type V monomère au moins étant **caractérisé en ce qu'**au moins une structure en boucle CDR ou une partie de celle-ci est modifiée ou remplacée de telle manière que
(i) la dimension de la structure CDR est augmentée quand elle est comparée avec la structure en boucle CDR correspondante dans le VLD non modifié; et/ou
(ii) la modification ou le remplacement donne comme résultat la formation d'une liaison disulfure à l'intérieur ou entre une ou plusieurs des structures en boucle CDR.

2. Fraction de fixation selon la revendication 1, dans laquelle la dimension de la structure en boucle CDR est augmentée par un résidu d'acide aminé.

3. Fraction de fixation selon la revendication 1, dans laquelle la dimension de la structure en boucle CDR est augmentée par au moins deux structures d'acide aminé.

4. Fraction de fixation selon la revendication 1, dans laquelle la dimension de la structure en boucle CDR est augmentée d'au moins six structures d'acide aminé.

5. Fraction de fixation selon la revendication 1, dans laquelle la dimension de la structure en boucle CDR est augmentée d'au moins neufs résidus d'acide aminé.

6. Fixation selon l'une quelconque des revendications 1 à 5 dans laquelle la solubilité du VLD modifié est améliorée quand elle est comparée au VLD non modifié.

7. Fraction de fixation selon l'une quelconque des revendications 1 à 6 dans laquelle l'affinité de fixation du VLD modifié est altérée quand elle est comparée avec le VLD non modifié.

8. Fraction de fixation selon la revendication 7 dans laquelle l'affinité du VLD modifié par rapport à au moins un ligand naturel du VLD non modifié est réduite.

9. Fraction de fixation selon l'une quelconque des revendications 1 à 8 dans laquelle la spécificité de fixation du VLD modifié est différente de celle du VLD non modifié.

10. Fraction de fixation selon l'une quelconque des revendications 1 à 9, dans laquelle le ligand non anticorps est une protéine de surface à cellule T.

11. Fraction de fixation selon la revendication 10, dans laquelle le non anticorps est CTLA-4, CD28 ou ICOS.

12. Fraction de fixation selon la revendication 11, dans laquelle le ligand non anticorps est CTLA-4.

13. Fraction de fixation selon l'une quelconque des revendications 1 à 12, dans laquelle une ou plusieurs des structures en boucle CDR est remplacée par un déterminant de fixation dérivé d'un polypeptide non anticorps.

14. Fraction de fixation selon la revendication 13, dans laquelle le déterminant de fixation est dérivé de la somatostatine ou l'haemagglutinine.

15. Fraction de fixation selon l'une quelconque des revendications 1 à 12, dans laquelle une ou plusieurs des structures en boucle CDR est remplacée par une ou plusieurs structures en boucle CDR dérivées d'un anticorps ou d'anticorps.

16. Fraction de fixation selon la revendication 15, dans laquelle l'anticorps ou les anticorps sont dérivés d'un rat, d'une souris, d'un humain, d'un chameau, d'un lama ou d'un requin.

17. Fraction de fixation selon la revendication 15 ou la revendication 16, dans laquelle l'anticorps ou les anticorps sont sélectionnés parmi l'anticorps du chameau cAB-Lys3 et l'anticorps anti-mélanome humain V86.

18. Fraction de fixation selon l'une quelconque des revendications 1 à 17, liée à un réactif diagnostique.

19. Fraction de fixation selon la revendication 18, dans laquelle le réactif diagnostique est sélectionné parmi le groupe constitué des streptavidine, biotine, un radioisotope, un colorant marqueur ou un autre réactif d'imagerie.

20. Réactif comprenant deux fractions de fixation ou plus selon l'une quelconque des revendications précédentes couplées ensemble soit chimiquement soit génétiquement.

21. Fraction de fixation ou réactif selon l'une quelconque des revendications 1 à 20 immobilisée sur un support solide ou couplée à une surface de biocapteur.

22. Polynucléotide codant une fraction de fixation ou un réactif selon l'une quelconque des revendications 1 à 20.

23. Vecteur comprenant un polynucléotide selon la revendication 22.

24. Cellule hôte comprenant un vecteur selon la revendication 23.

25. Cellule hôte selon la revendication 24 dans laquelle la cellule est une cellule bactérienne.

26. Procédé de production d'une fraction de fixation qui comprend la culture d'une cellule hôte selon la revendication 24 ou la revendication 25 dans des conditions favorisant l'expression de la fraction de fixation et facultativement la récupération de la fraction de fixation.

27. Procédé selon la revendication 26 dans lequel la fraction de fixation est non glycosylée.

28. Composition pharmaceutique comprenant une fraction de fixation selon l'une quelconque des revendications 1 à 20 et un support ou diluant acceptable sur le plan pharmaceutique.

29. Fraction de fixation ou réactif selon l'une quelconque des revendications 1 à 20 à utiliser dans le traitement d'un état pathologique dans un sujet.

30. Procédé de sélection d'une fraction de fixation avec une affinité pour une molécule cible qui comprend le criblage d'une bibliothèque de polynucléotides pour l'expression d'une fraction de fixation avec une affinité pour la molécule cible, les polynucléotides codant un ou plusieurs VLD ligand non anticorps, dans lequel les polynucléotides ont été soumis à une mutagenèse qui résulte dans une modification ou le remplacement dans au moins une structure en boucle CDR dans au moins un VLD de sorte que
(i) la dimension de la structure en boucle CDR augmente comparée à la structure en boucle CDR correspondante dans le VLD non modifié, et/ou
(ii) la modification ou le remplacement donne comme résultat la formation d'une liaison disulfure à l'intérieur ou entre une ou plusieurs des structures en boucle CDR.

31. Procédé selon la revendication 30 dans lequel la solubilité du VLD modifié isolé est améliorée quand elle est comparée au VLD non modifié isolé.

32. Procédé selon la revendication 31 ou la revendication 32, dans lequel le processus de criblage implique l'affichage des domaines de type V modifiés comme fusions de protéine de gène III à la surface de particules bactériophages.

33. Procédé selon la revendication 31 ou la revendication 32, dans lequel le processus de criblage implique l'affichage des domaines de type V modifiés dans un système de sélection d'affichage ribosomal.


### SEQUENCE LISTING

<110> Diatech Pty Ltd
<120> V-like domain binding molecules
<150> PP2210
   <151> 1998-03-06
<160> 138
<170> PatentIn Ver. 2.1
<210> 1
   <211> 6
   <212> PRT
   <213> Homo sapiens
<400> 1
   Met Tyr Pro Pro Pro Tyr
   1 5
<210> 2
   <211> 54
   <212> DNA
   <213> Artificial Sequence
<220>
   <223> Description of Artificial Sequence Oligonucleotide PCR primer
<400> 2
   ttattactcg cggcccagcc ggccatggcc gcaatgcacg tggcccagcc tgct 54
<210> 3
   <211> 60
   <212> DNA
   <213> Artificial Sequence
<220>
   <223> Description of Artificial Sequence Oligonucleotide PCR primer
<400> 3
   ttattactcg cggcccagcc ggccatggcc gcaatgcacg tggcccagcc tgctgtggta 60
<210> 4
   <211> 45
   <212> DNA
   <213> Artificial Sequence
<220>
   <223> Description of Artificial Sequence Oligonucleotide PCR primer
<400> 4
   tctcacagtg cacaggcaat gcacgtggcc cagcctgctg tggta 45
<210> 5
   <211> 39
   <212> DNA
   <213> Artificial Sequence
<220>
   <223> Description of Artificial Sequence Oligonucleotide PCR primer
<400> 5
   tctcacagtg cacaggcaat gcacgtggcc cagcctgct 39
<210> 6
   <211> 42
   <212> DNA
   <213> Artificial Sequence
<220>
   <223> Description of Artificial Sequence Oligonucleotide PCR primer
<400> 6
   gcccagccgg ccgaattcgc aatgcacgtg gcccagcctg ct 42
<210> 7
   <211> 60
   <212> DNA
   <213> Artificial Sequence
<220>
   <223> Description of Artificial Sequence Oligonucleotide PCR primer
<400> 7
   gcagctaata cgactcacta taggaacaga ccaccatgga cgtggcccag cctgctgtgg 60
<210> 8
   <211> 42
   <212> DNA
   <213> Artificial Sequence
<220>
   <223> Description of Artificial Sequence Oligonucleotide PCR primer
<400> 8
   atctgcggcc gctacataaa tctgggtacc gttgccgatg cc 42
<210> 9
   <211> 66
   <212> DNA
   <213> Artificial Sequence
<220>
   <223> Description of Artificial Sequence Oligonucleotide PCR primer
<400> 9
   gctgaattct gatcagtgat ggtgatggtg atgtgcggcc gcgtcagaat ctgggcacgg 60
   ttctgg 66
<210> 10
   <211> 51
   <212> DNA
   <213> Artificial Sequence
<220>
   <223> Description of Artificial Sequence Oligonucleotide PCR primer
<400> 10
   gcccttgggc cgggagatgg tctgcttcag tggcgagggc aggtctgtgt g 51
<210> 11
   <211> 49
   <212> DNA
   <213> Artificial Sequence
<220>
   <223> Description of Artificial Sequence Oligonucleotide PCR primer
<400> 11
   cgagggcagg tctgtgtggg tcacggtgca cgtgaacctc tccccggag 49
<210> 12
   <211> 51
   <212> DNA
   <213> Artificial Sequence
<220>
   <223> Description of Artificial Sequence Oligonucleotide PCR primer
<400> 12
   cgtgaacctc tccccggagt tccagtcatc ctcgcagatg ctggcctcac c 51
<210> 13
   <211> 84
   <212> DNA
   <213> Artificial Sequence
<220>
   <223> Description of Artificial Sequence Oligonucleotide PCR primer
<400> 13
   agctttgtgt gtgagtatgc agctggctgc aagaatttct tctggaagac tttcacatcc 60
   tgtgccactg aggtccgggt gaca 84
<210> 14
   <211> 84
   <212> DNA
   <213> Artificial Sequence
<220>
   <223> Description of Artificial Sequence Oligonucleotide PCR primer
<400> 14
   ctgggtaccg ttgccgatgc cacaggatgt gaaagtcttc cagaagaaat tcttgcagcc 60
   agcctccacc ttgcagatgt agag 84
<210> 15
   <211> 75
   <212> DNA
   <213> Artificial Sequence
<220>
   <223> Description of Artificial Sequence Oligonucleotide PCR primer
<400> 15
   agctttgtgt gtgagtatgc agctggctgc aagaatnnkn nknnknnknn knnkacatcc 60
   tgtgccactg aggtc 75
<210> 16
   <211> 75
   <212> DNA
   <213> Artificial Sequence
<220>
   <223> Description of Artificial Sequence Oligonucleotide PCR primer
<400> 16
   ctgggtaccg ttgccgatgc cacaggatgt mnnmnnmnnm nnmnnmnnat tcttgcagcc 60
   agcctccacc ttgca 75
<210> 17
   <211> 21
   <212> DNA
   <213> Artificial Sequence
<220>
   <223> Description of Artificial Sequence Oligonucleotide PCR primer
<400> 17
   gtaggttgcc gcacagactt c 21
<210> 18
   <211> 65
   <212> DNA
   <213> Artificial Sequence
<220>
   <223> Description of Artificial Sequence Oligonucleotide PCR primer
<400> 18
   gaagtctgtg cggcaaccta cccgtatgac gttccgacta cgccctagat gattccatct 60
   gcacg 65
<210> 19
   <211> 78
   <212> DNA
   <213> Artificial Sequence
<220>
   <223> Description of Artificial Sequence Oligonucleotide PCR primer
<400> 19
   gccagctttg tgtgtgagta tgccagtggc tacaccatcg ggccgtactg catgggcgtc 60
   cgggtgacag tgcttcgg 78
<210> 20
   <211> 60
   <212> DNA
   <213> Artificial Sequence
<220>
   <223> Description of Artificial Sequence Oligonucleotide PCR primer
<400> 20
   tgtgcggcag ccatcaacat gggcggtggc atcaccttcc tagatgattc catctgcacg 60
<210> 21
   <211> 60
   <212> DNA
   <213> Artificial Sequence
<220>
   <223> Description of Artificial Sequence Oligonucleotide PCR primer
<400> 21
   atctaggaag gtgatgccac cgcccatgtt gatggctgcc gcacagactt cagtcacctg 60
<210> 22
   <211> 69
   <212> DNA
   <213> Artificial Sequence
<220>
   <223> Description of Artificial Sequence Oligonucleotide PCR primer
<400> 22
   cagcccgtgg ccgcactcgt agtaggacgc gtagatcgtc gagtccacct tgcagatgta 60
   gagtcccgt 69
<210> 23
   <211> 72
   <212> DNA
   <213> Artificial Sequence
<220>
   <223> Description of Artificial Sequence Oligonucleotide PCR primer
<400> 23
   aatctgggta ccgttgccga tgccggagtc atagccgtac cctcccgtgg acagcccgtg 60
   gccgcactcg ta 72
<210> 24
   <211> 78
   <212> DNA
   <213> Artificial Sequence
<220>
<223> Description of Artificial Sequence
   Oligonucleotide PCR primer
<400> 24
   gccagctttg tgtgtgagta tgccagtgga ttcaccttca gttcctacgc catgtccgtc 60
   cgggtgacag tgcttcgg 78
<210> 25
   <211> 51
   <212> DNA
   <213> Artificial Sequence
<220>
   <223> Description of Artificial Sequence Oligonucleotide PCR primer
<400> 25
   gccatctccg gatccggagg ctcgacctac ctagatgatt ccatctgcac g 51
<210> 26
   <211> 54
   <212> DNA
   <213> Artificial Sequence
<220>
   <223> Description of Artificial Sequence Oligonucleotide PCR primer
<400> 26
   gtaggtcgag cctccggatc cggagatggc tgccgcacag acttcagtca cctg 54
<210> 27
   <211> 69
   <212> DNA
   <213> Artificial Sequence
<220>
   <223> Description of Artificial Sequence Oligonucleotide PCR primer
<400> 27
   cacgtccatg tagtagtctc cctcctcgcc gcgcagtccc cagcccacct tgcagatgta 60
   gagtcccgt 69
<210> 28
   <211> 51
   <212> DNA
   <213> Artificial Sequence
<220>
   <223> Description of Artificial Sequence Oligonucleotide PCR primer
<400> 28
   aatctgggta ccgttgccga tgcccacgtc catgtagtag tctccctcct c 51
<210> 29
   <211> 66
   <212> DNA
   <213> Artificial Sequence
<220>
   <223> Description of Artificial Sequence Oligonucleotide PCR primer
<400> 29
   agctttgtgt gtgagtatgc annknnknnk nnknnknnkn nknnkgccac tgaggtccgg 60
   gtgaca                                                            66
<210> 30
   <211> 68
   <212> DNA
   <213> Artificial Sequence
<220>
   <223> Description of Artificial Sequence Oligonucleotide PCR primer
<400> 30
   cacgtggccc agcctgctgt ggtactggcc agcagccgag gcatcgccag ctttgtgtgt 60
   gagtatgc                                                          68
<210> 31
   <211> 66
   <212> DNA
   <213> Artificial Sequence
<220>
   <223> Description of Artificial Sequence Oligonucleotide PCR primer
<400> 31
   gtgtgtgagt acgcgtncnn snnsnnsnns nnsnnstgcn nsgctactga ggttcgtgtg 60
   accgtc                                                            66
<210> 32
   <211> 73
   <212> DNA
   <213> Artificial Sequence
<220>
   <223> Description of Artificial Sequence Oligonucleotide PCR primer
<400> 32
   gccagctttg tgtgtgagta tgcannknnk nnknnknnkn nknnkggcgt ccgggtgaca 60
   gtgcttcggc agg                                                    73
<210> 33
   <211> 82
   <212> DNA
   <213> Artificial Sequence
<220>
   <223> Description of Artificial Sequence Oligonucleotide PCR Primer
<400> 33
   gccagctttg tgtgtgagta tgcannknnk nnknnknnkn nknnknnktg cnnkggcgtc 60
   cgggtgacag tgcttcggca gg 82
<210> 34
   <211> 82
   <212> DNA
   <213> Artificial Sequence
<220>
   <223> Description of Artificial Sequence Oligonucleotide PCR Primer
<400> 34
   gccagctttg tgtgtgagta tgcannknnk ywynnkywyn nknnkywytg cnnkggcgtc 60
   cgggtgacag tgcttcggca gg 82
<210> 35
   <211> 70
   <212> DNA
   <213> Artificial Sequence
<220>
   <223> Description of Artificial Sequence Oligonucleotide PCR primer
<400> 35
   gccagctttg tgtgtgagta tgcatctcca ggcnnknnkn nknnkgtccg ggtgacagtg 60
   cttcggcagg 70
<210> 36
   <211> 70
   <212> DNA
   <213> Artificial Sequence
<220>
   <223> Description of Artificial Sequence Oligonucleotide PCR primer
<400> 36
   gccagctttg tgtgtgagta tgcatctcca ggcnnktgcn nknnkgtccg ggtgacagtg 60
   cttcggcagg 70
<210> 37
   <211> 67
   <212> DNA
   <213> Artificial Sequence
<220>
   <223> Description of Artificial Sequence Oligonucleotide PCR primer
<400> 37
   tgactgaagt ctgtgcggca acctacnnkn nkgggnnkga gttgaccttc ctagatgatt 60
   ccatctg 67
<210> 38
   <211> 30
   <212> DNA
   <213> Artificial Sequence
<220>
   <223> Description of Artificial Sequence Oligonucleotide PCR primer
<400> 38
   gtaggttgcc gcacagactt cagtcacctg 30
<210> 39
   <211> 68
   <212> DNA
   <213> Artificial Sequence
<220>
   <223> Description of Artificial Sequence Oligonucleotide PCR primer
<400> 39
   gtgactgaag tctgtgcggc atgctacnnk nnkgggnnkg agttgacctt cctagatgat 60
   tccatctg 68
<210> 40
   <211> 29
   <212> DNA
   <213> Artificial Sequence
<220>
   <223> Description of Artificial Sequence Oligonucleotide PCR primer
<400> 40
   tagcatgccg cacagacttc agtcacctg 29
<210> 41
   <211> 69
   <212> DNA
   <213> Artificial Sequence
<220>
   <223> Description of Artificial Sequence Oligonucleotide PCR primer
<400> 41
   ctgggtaccg ttgccgatgc cmnnmnnmnn mnnmnnmnnm nnmnnmnnct ccaccttgca 60
   gatgtagag 69
<210> 42
   <211> 67
   <212> DNA
   <213> Artificial Sequence
<22C>
   <223> Description of Artificial Sequence Oligonucleotide PCR primer
<400> 42
   aggtggaann snnsnnsnns nnsnnstgcn nsnnsnnsnn snnsnnsnns ggcatcggca 60
   acggtac 67
<210> 43
   <211> 78
   <212> DNA
   <213> Artificial Sequence
<220>
   <223> Description of Artificial Sequence Oligonucleotide PCR primer
<400> 43
   aatctgggta ccgttgccga tgccmnnmnn mnnmnnmnnm nnmnnmnnmn nmnncacctt 60
   gcagatgtag agtcccgt 78
<210> 44
   <211> 93
   <212> DNA
   <213> Artificial Sequence
<220>
   <223> Description of Artificial Sequence Oligonucleotide PCR primer
<400> 44
   aatctgggta ccgttgccga tgcccagmnn mnnmnnmnnm nnmnnmnnmn nmnnmnnmnn 60
   mnnmnnctcc accttgcaga tgtagagtcc cgt 93
<210> 45
   <211> 81
   <212> DNA
   <213> Artificial Sequence
<220>
   <223> Description of Artificial Sequence Oligonucleotide PCR primer
<400> 45
   aatctgggta ccgttgccga tgccmnnmnn mnnmnngcam nnmnnmnnmn nmnnmnncac 60
   cttgcagatg tagagtcccg t 81
<210> 46
   <211> 87
   <212> DNA
   <213> Artificial Sequence
<220>
   <223> Description of Artificial Sequence Oligonucleotide PCR primer
<400> 46
   aatctgggta ccgttgccga tgccmnnmnn mnnmnnmnng camnnmnnmn nmnnmnnmnn 60
   mnncaccttg cagatgtaga gtcccgt                                      87
<210> 47
   <211> 99
   <212> DNA
   <213> Artificial Sequence
<220>
   <223> Description of Artificial Sequence Oligonucleotide PCR primer
<400> 47
   aatctgggta ccgttgccga tgccmnnmnn mnnmnnmnnm nngcamnnmn nmnnmnnmnn 60
   mnnmnnmnnm nnmnncacct tgcagatgta gagtcccgt                         99
<210> 48
   <211> 87
   <212> DNA
   <213> Artificial Sequence
<220>
   <223> Description of Artificial Sequence Oligonucleotide PCR primer
<400> 48
   aatctgggta ccgttgccga tgccrwrmnn mnnmnnmnng camnnmnnmn nmnnmnnmnn 60
   mnncaccttg cagatgtaga gtcccgt                                      87
<210> 49
   <211> 70
   <212> DNA
   <213> Artificial Sequence
<220>
   <223> Description of Artificial Sequence Oligonucleotide PCR primer
<400> 49
   atgcacgtgg cccagcctgc tgtggtgctg gccagcagcc gtggcatcgc cagctttgtg 60
   tgtgaatatg                                                         70
<210> 50
   <211> 77
   <212> DNA
   <213> Artificial Sequence
<220>
   <223> Description of Artificial Sequence Oligonucleotide PCR primer
<400> 50
   gccagctttg tgtgtgaata tgcgtctggc tataccatcg gcccgtactg catgggtgtg 60
   cgtgtgaccg tgctgcg 77
<210> 51
   <211> 54
   <212> DNA
   <213> Artificial Sequence
<220>
   <223> Description of Artificial Sequence Oligonucleotide PCR primer
<400> 51
   gtgcgtgtga ccgtgctgcg tcaggcggat agccaggtga ccgaagtttg cgcg 54
<210> 52
   <211> 75
   <212> DNA
   <213> Artificial Sequence
<220>
   <223> Description of Artificial Sequence Oligonucleotide PCR primer
<400> 52
   caggtgaccg aagtttgcgc ggcagcgatc aacatgggcg gtggcatcac cttcctggat 60
   gattccatct gcacc 75
<210> 53
   <211> 66
   <212> DNA
   <213> Artificial Sequence
<220>
   <223> Description of Artificial Sequence Oligonucleotide PCR primer
<400> 53
   cagaccctgg atggtcaggt tcacctggtt accgctggag gtgccggtgc agatggaatc 60
   atccag 66
<210> 54
   <211> 57
   <212> DNA
   <213> Artificial Sequence
<220>
   <223> Description of Artificial Sequence Oligonucleotide PCR primer
<400> 54
   cactttgcag atgtacagac cggtatccat ggcacgcaga ccctggatgg tcaggtt 57
<210> 55
   <211> 66
   <212> DNA
   <213> Artificial Sequence
<220>
   <223> Description of Artificial Sequence Oligonucleotide PCR primer
<400> 55
   caggccatga ccgcattcgt aataagacgc atagatggtg ctatccactt tgcagatgta 60
   cagacc 66
<210> 56
   <211> 69
   <212> DNA
   <213> Artificial Sequence
<220>
   <223> Description of Artificial Sequence Oligonucleotide PCR primer
<400> 56
   ctgggtaccg ttgccgatgc cagaatcgta gccatagcca ccggtggaca ggccatgacc 60
   gcattcgta 69
<210> 57
   <211> 672
   <212> DNA
   <213> Homo sapiens
<400> 57
   atggcttgcc ttggatttca gcggcacaag gctcagctga acctggctgc caggacctgg 60
   ccctgcactc tcctgttttt tcttctcttc atccctgtct tctgcaaagc aatgcacgtg 120
   gcccagcctg ctgtggtact ggccagcagc cgaggcatcg ccagctttgt gtgtgagtat 180
   gcatctccag gcaaagccac tgaggtccgg gtgacagtgc ttcggcaggc tgacagccag 240
   gtgactgaag tctgtgcggc aacctacatg acggggaatg agttgacctt cctagatgat 300
   tccatctgca cgggcacctc cagtggaaat caagtgaacc tcactatcca aggactgagg 360
   gccatggaca cgggactcta catctgcaag gtggagctca tgtacccacc gccatactac 420
   ctgggcatag gcaacggaac ccagatttat gtaattgatc cagaaccgtg cccagattct 480
   gacttcctcc tctggatcct tgcagcagtt agttcggggt tgttttttta tagctttctc 540
   ctcacagctg tttctttgag caaaatgcta aagaaaagaa gccctcttac aacaggggtc 600
   tatgtgaaaa tgcccccaac agagccagaa tgtgaaaagc aatttcagcc ttattttatt 660
   cccatcaatt ga 672
<210> 58
   <211> 115
   <212> PRT
   <213> Homo sapiens
<400> 58
   Ala Met His Val Ala Gln Pro Ala Val Val Leu Ala Ser Ser Arg Gly
    1               5                  10                  15
   Ile Ala Ser Phe Val Cys Glu Tyr Ala Ser Pro Gly Lys Ala Thr Glu
               20                  25                  30
   Val Arg Val Thr Val Leu Arg Gln Ala Asp Ser Gln Val Thr Glu Val
           35                  40                  45
   Cys Ala Ala Thr Tyr Met Met Gly Asn Glu Leu Thr Phe Leu Asp Asp
       50                  55                  60
   Ser Ile Cys Thr Gly Thr Ser Ser Gly Asn Gln Val Asn Leu Thr Ile
   65                  70                  75                  80
   Gln Gly Leu Arg Ala Met Asp Thr Gly Leu Tyr Ile Cys Lys Val Glu
                   85                  90                  95
   Leu Met Tyr Pro Pro Pro Tyr Tyr Leu Gly Ile Gly Asn Gly Ala Gln
               100                 105                 110
   Ile Tyr Val
           115
<210> 59
   <211> 7
   <212> PRT
   <213> Homo sapiens
<400> 59
   Ser Pro Gly Lys Ala Thr Glu
    1               5
<210> 60
   <211> 9
   <212> PRT
   <213> Homo sapiens
<400> 60
   Tyr Met Met Gly Asn Glu Leu Thr Phe
    1               5
<210> 61
   <211> 9
   <212> PRT
   <213> Homo sapiens
<400> 61
   Leu Met Tyr Pro Pro Pro Tyr Tyr Leu
   1 5
<210> 62
   <211> 14
   <212> PRT
   <213> Homo sapiens
<400> 62
   Ala Gly Cys Lys Asn Phe Phe Trp Lys Thr Phe Thr Ser Cys
   1 5 10
<210> 63
   <211> 9
   <212> PRT
   <213> Artificial Sequence
<220>
   <223> Description of Artificial Sequence Haemagglutinin tag
<400> 63
   Tyr Pro Tyr Asp Val Pro Asp Tyr Ala
   1 5
<210> 64
   <211> 11
   <212> PRT
   <213> Artificial Sequence
<220>
   <223> Description of Artificial Sequence Sequence from anti-lysozyme antibody
<400> 64
   Ser Gly Tyr Thr Ile Gly Pro Tyr Cys Met Gly
   1 5 10
<210> 65
   <211> 10
   <212> PRT
   <213> Artificial Sequence
<220>
   <223> Description of Artificial Sequence Sequence from anti-lysozyme antibody
<400> 65
   Thr Tyr Met Met Gly Asn Glu Leu Thr Phe
   1 5 10
<210> 66
   <211> 24
   <212> PRT
   <213> Artificial Sequence
<220>
   <223> Description of Artificial-Sequence Sequence from anti-lysozyme antibody
<400> 66
   Asp Ser Thr Ile Tyr Ala Ser Tyr Tyr Glu Cys Gly His Gly Leu Ser
   1 5 10 15
   Thr Gly Gly Tyr Gly Tyr Asp Ser
   20
<210> 67
   <211> 11
   <212> PRT
   <213> Homo sapiens
<400> 67
   Ser Gly Phe Thr Phe Ser Ser Tyr Ala Met Ser
   1 5 10
<210> 68
   <211> 10
   <212> PRT
   <213> Homo sapiens
<400> 68
   Ala Ile Ser Gly Ser Gly Gly Ser Thr Tyr
   1 5 10
<210> 69
   <211> 15
   <212> PRT
   <213> Homo sapiens
<400> 69
   Gly Trp Gly Leu Arg Gly Glu Glu Gly Asp Tyr Tyr Met Asp Val
   1 5 10 15
<210> 70
   <211> 21
   <212> PRT
   <213> Artificial Sequence
<220>
   <223> Description of Artificial Sequence Flag-tag
<400> 70
   Ala Ala Ala Asp Tyr Lys Asp Asp Asp Asp Lys Ala Ala Asp Tyr Lys
   1 5 10 15
   Asp Asp Asp Asp Lys
   20
<210> 71
   <211> 14
   <212> PRT
   <213> Homo sapiens
<400> 71
   Ser Phe Val Cys Glu Tyr Ala Ser Pro Gly Lys Ala Thr Glu
   1 5 10
<210> 72
   <211> 18
   <212> PRT
   <213> Artificial Sequence
<220>
   <223> Description of Artificial Sequence Fusion protein
<400> 72
   Ser Phe Val Cys Glu Tyr Ala Ser Gly Tyr Thr Ile Gly Pro Tyr Cys
   1 5 10 15
   Met Gly
<210> 73
   <211> 24
   <212> PRT
   <213> Artificial Sequence
<220>
   <223> Description of Artificial Sequence Fusion protein
<400> 73
   Ser Phe Val Cys Glu Tyr Ala Ala Gly Cys Lys Asn Phe Phe Trp Lys
   1 5 10 15
   Thr Phe Thr Ser Cys Ala Thr Glu
   20
<210> 74
   <211> 18
   <212> PRT
   <213> Artificial Sequence
<220>
   <223> Description of Artificial Sequence Fusion protein
<400> 74
   Ser Phe Val Cys Glu Tyr Ala Ser Gly Phe Thr Phe Ser Ser Tyr Ala
   1 5 10 15
   Met Ser
<210> 75
   <211> 15
   <212> PRT
   <213> Artificial Sequence
<220>
   <223> Description of Artificial Sequence Fusion protein containing random sequence
<400> 75
   Ser Phe Val Cys Glu Tyr Ala Xaa Xaa Xaa Xaa Xaa Xaa Xaa Gly
   1 5 10 15
<210> 76
   <211> 18
   <212> PRT
   <213> Artificial Sequence
<220>
   <223> Description of Artificial Sequence Fusion protein containing random sequence
<400> 76
   Ser Phe Val Cys Glu Tyr Ala Xaa Xaa Xaa Xaa Xaa Xaa Xaa Xaa Cys
   1 5 10 15
   Xaa Gly
<210> 77
   <211> 21
   <212> PRT
   <213> Artificial Sequence
<220>
   <223> Description of Artificial Sequence Fusion protein containing random sequence
<400> 77
   Ser Phe Val Cys Glu Tyr Ala Xaa Xaa Ala Arg Xaa Ala Arg Xaa Xaa
   1 5 10 15
   Ala Arg Cys Xaa Gly
   20
<210> 78
   <211> 14
   <212> PRT
   <213> Artificial Sequence
<220>
   <223> Description of Artificial Sequence Fusion protein containing random sequence
<400> 78
   Ser Phe Val Cys Glu Tyr Ala Ser Pro Gly Xaa Xaa Xaa Xaa
   1 5 10
<210> 79
   <211> 14
   <212> PRT
   <213> Artificial Sequence
<220>
   <223> Description of Artificial Sequence Fusion protein containing random sequence
<400> 79
   Ser Phe Val Cys Glu Tyr Ala Ser Pro Gly Xaa Cys Xaa Xaa
   1 5 10
<210> 80
   <211> 18
   <212> PRT
   <213> Artificial Sequence
<220>
   <223> Description of Artificial Sequence Fusion protein containing random sequence
<400> 80
   Ser Phe Val Cys Glu Tyr Ala Xaa Xaa Xaa Xaa Xaa Xaa Xaa Xaa Ala
   1 5 10 15
   Thr Glu
<210> 81
   <211> 18
   <212> PRT
   <213> Artificial Sequence
<220>
   <223> Description of Artificial Sequence Fusion protein containing random sequence
<400> 81
   Ser Phe Val Cys Glu Tyr Ala Xaa Xaa Xaa Xaa Xaa Xaa Cys Xaa Ala
   1 5 10 15
   Thr Glu
<210> 82
   <211> 24
   <212> PRT
   <213> Artificial Sequence
<220>
   <223> Description of Artificial Sequence Fusion protein containing random sequence
<400> 82
   Ser Phe Val Cys Glu Tyr Ala Ala Gly Cys Lys Asn Xaa Xaa Xaa Xaa
   1 5 10 15
   Xaa Xaa Thr Ser Cys Ala Thr Glu
   20
<210> 83
   <211> 25
   <212> PRT
   <213> Homo sapiens
<400> 83
   Gln Val Thr Glu Val Cys Ala Ala Thr Tyr Met Met Gly Asn Glu Leu
   1 5 10 15
   Thr Phe Leu Asp Asp Ser Ile Cys Thr
   20 25
<210> 84
   <211> 25
   <212> PRT
   <213> Artificial Sequence
<220>
   <223> Description of Artificial Sequence Fusion protein
<400> 84
   Gln Val Thr Glu Val Cys Ala Ala Ala Ile Asn Met Gly Gly Gly Ile
   1               5                   10                  15
   Thr Phe Leu Asp Asp Ser Ile Cys Thr
               20                  25
<210> 85
   <211> 25
   <212> PRT
   <213> Artificial Sequence
<220>
   <223> Description of Artificial Sequence Fusion protein
<400> 85
   Gln Val Thr Glu Val Cys Ala Ala Thr Tyr Pro Tyr Asp Val Pro Asp
   1               5                   10                  15
   Tyr Ala Leu Asp Asp Ser Ile Cys Thr
               20                  25
<210> 36
   <211> 25
   <212> PRT
   <213> Artificial Sequence
<220>
   <223> Description of Artificial Sequence Fusion protein
<400> 86
<210> 87
   <211> 25
   <212> PRT
   <213> Artificial Sequence
<220>
   <223> Description of Artificial Sequence Fusion protein containing random sequence
<400> 87
   Gln Val Thr Glu Val Cys Ala Ala Thr Tyr Xaa Xaa Gly Xaa Glu Leu
    1               5                  10                  15
   Thr Phe Leu Asp Asp Ser Ile Cys Thr
               20                  25
<210> 88
   <211> 25
   <212> PRT
   <213> Artificial Sequence
<220>
   <223> Description of Artificial Sequence Fusion protein containing random sequence
<400> 88
   Gln Val Thr Glu Val Cys Ala Ala Cys Tyr Xaa Xaa Gly Xaa Glu Leu
    1               5                  10                  15
   Thr Phe Leu Asp Asp Ser Ile Cys Thr
               20                  25
<210> 89
   <211> 13
   <212> PRT
   <213> Homo sapiens
<400> 89
   Cys Lys Val Glu Leu Met Tyr Pro Pro Pro Tyr Tyr Leu
    1               5                  10
<210> 90
   <211> 27
   <212> PRT
   <213> Artificial Sequence
<220>
   <223> Description of Artificial Sequence Fusion protein
<400> 90
   Cys Lys Val Asp Ser Thr Ile Tyr Ala Ser Tyr Tyr Glu Cys Gly His
   1 5 10 15
   Gly Leu Ser Thr Gly Gly Tyr Gly Tyr Asp Ser
   20 25
<210> 91
   <211> 18
   <212> PRT
   <213> Artificial Sequence
<220>
   <223> Description of Artificial Sequence Fusion protein
<400> 91
   Cys Lys Val Glu Ala Gly Cys Lys Asn Phe Phe Trp Lys Thr Phe Thr
   1 5 10 15
   Ser Cys
<210> 92
   <211> 18
   <212> PRT
   <213> Artificial Sequence
<220>
   <223> Description of Artificial Sequence Fusion protein
<400> 92
   Cys Lys Val Gly Trp Gly Leu Arg Gly Glu Glu Gly Asp Tyr Tyr Met
   1 5 10 15
   Asp Val
<210> 93
   <211> 14
   <212> PRT
   <213> Artificial Sequence
<220>
   <223> Description of Artificial Sequence Fusion protein containing random sequence
<400> 93
   Cys Lys Val Xaa Xaa Xaa Xaa Xaa Xaa Xaa Xaa Xaa Xaa Xaa
   1 5 10
<210> 94
   <211> 18
   <212> PRT
   <213> Artificial Sequence
<220>
   <223> Description of Artificial Sequence Fusion protein containing random sequence
<400> 94
   Cys Lys Val Xaa Xaa Xaa Xaa Xaa Xaa Xaa Xaa Xaa Xaa Xaa Xaa Xaa
   1 5 10 15
   Xaa Xaa
<210> 95
   <211> 14
   <212> PRT
   <213> Artificial Sequence
<220>
   <223> Description of Artificial Sequence Fusion protein containing random sequence
<400> 95
   Cys Lys Val Xaa Xaa Xaa Xaa Xaa Xaa Cys Xaa Xaa Xaa Xaa
   1 5 10
<210> 96
   <211> 15
   <212> PRT
   <213> Artificial Sequence
<220>
   <223> Description of Artificial Sequence Fusion protein containing random sequence
<400> 96
   Cys Lys Val Xaa Xaa Xaa Xaa Xaa Xaa Xaa Cys Xaa Xaa Xaa Xaa
   1 5 10 15
<210> 97
   <211> 17
   <212> PRT
   <213> Artificial Sequence
<220>
   <223> Description of Artificial Sequence Fusion protein containing random sequence
<400> 97
   Cys Lys Val Xaa Xaa Xaa Xaa Xaa Xaa Xaa Xaa Cys Xaa Xaa Xaa Xaa
   1 5 10 15
   Xaa
<210> 98
   <211> 21
   <212> PRT
   <213> Artificial Sequence
<220>
   <223> Description of Artificial Sequence Fusion protein containing random sequence
<400> 98
   Cys Lys Val Xaa Xaa Xaa Xaa Xaa Xaa Xaa Xaa Xaa Xaa Cys Xaa Xaa
   1 5 10 15
   Xaa Xaa Xaa Xaa Xaa
   20
<210> 99
   <211> 13
   <212> PRT
   <213> Artificial Sequence
<220>
   <223> Description of Artificial Sequence Fusion protein containing random sequence
<400> 99
   Cys Lys Val Glu Xaa Xaa Xaa Xaa Xaa Xaa Xaa Xaa Xaa
   1 5 10
<210> 100
   <211> 18
   <212> PRT
   <213> Artificial Sequence
<220>
   <223> Description of Artificial Sequence Fusion protein containing random sequence
<400> 100
   Cys Lys Val Glu Xaa Xaa Xaa Xaa Xaa Xaa Cys Xaa Xaa Xaa Xaa Xaa
   1 5 10 15
   Xaa Xaa
<210> 101
   <211> 18
   <212> PRT
   <213> Artificial Sequence
<220>
   <223> Description of Artificial Sequence Fusion protein containing random sequence
<400> 101
   Cys Lys Val Glu Ala Gly Cys Lys Asn Xaa Xaa Xaa Xaa Xaa Xaa Thr
   1 5 10 15
   Ser Cys
<210> 102
   <211> 10
   <212> PRT
   <213> Artificial Sequence
<220>
   <223> Description of Artificial Sequence CDR1 and CDR3 inserts possessing randomly generated sequence
<400> 102
   Leu Pro Ser Ser Asp Thr Arg Ala Tyr Ser
   1 5 10
<210> 103
   <211> 8
   <212> PRT
   <213> Artificial Sequence
<220>
   <223> Description of Artificial Sequence CDR1 and CDR3 inserts possessing randomly generated sequence
<400> 103
   Gln Glu Ser Gly Gly Arg Pro Gly
   1 5
<210> 104
   <211> 10
   <212> PRT
   <213> Artificial Sequence
<220>
   <223> Description of Artificial Sequence CDR1 and CDR3 inserts possessing randomly generated sequence
<400> 104
   Leu Pro Arg Gly Pro Pro Leu Leu Ser Leu
   1 5 10
<210> 105
   <211> 7
   <212> PRT
   <213> Artificial Sequence
<220>
   <223> Description of Artificial Sequence CDR1 and CDR3 inserts possessing randomly generated sequence
<400> 105
   Ser Pro Gly Arg Cys Leu Asn
   1 5
<210> 106
   <211> 8
   <212> PRT
   <213> Artificial Sequence
<220>
   <221> MOD_RES
   <222> (5)
   <223> Stop codon but Glu when expressed in Tg-1 or JM109 strains of E.coli
<220>
   <223> Description of Artificial Sequence CDR1 and CDR3 inserts possessing randomly generated sequence
<400> 106
   Glu Trp Lys Arg Glu His Gly Gly
   1 5
<210> 107
   <211> 10
   <212> PRT
   <213> Artificial Sequence
<220>
   <223> Description of Artificial Sequence CDR1 and CDR3 inserts possessing randomly generated sequence
<400> 107
   Leu Cys Pro Gly Ala Cys Gly Cys Val Tyr
   1 5 10
<210> 108
   <211> 8
   <212> PRT
   <213> Artificial Sequence
<220>
   <221> MOD_RES
   <222> (4)
   <223> Stop codon but Glu when expressed in Tg-1 of JM109 E.coli strains
<220>
   <223> Description of Artificial Sequence CDR1 and CDR3 inserts possessing randomly generated sequence
<400> 108
   Asn Ser Gly Glu Asn Glu Gly Gly
   1 5
<210> 109
   <211> 8
   <212> PRT
   <213> Artificial Sequence
<220>
   <223> Description of Artificial Sequence CDR1 and CDR3 inserts possessing randomly generated sequence
<400> 109
   Asp Lys Pro Val Thr Lys Ser Gly
   1 5
<210> 110
   <211> 7
   <212> PRT
   <213> Artificial Sequence
<220>
   <221> MOD RES
   <222> (7)
   <223> Stop codon but Glu when expressed in Tg-1 or JM109 strains of E. coli
<220>
   <223> Description of Artificial Sequence CDR1 and CDR3 inserts possessing randomly generated sequence
<400> 110
   Ser Pro Gly Ala Cys Pro Glu
   1 5
<210> 111
   <211> 7
   <212> PRT
   <213> Artificial Sequence
<220>
   <223> Description of Artificial Sequence CDR1 and CDR3 inserts possessing randomly generated sequence
<400> 111
   Ser Pro Gly Lys Cys Asp Gln
   1 5
<210> 112
   <211> 7
   <212> PRT
   <213> Artificial Sequence
<220>
   <223> Description of Artificial Sequence CDR1 and CDR3 inserts possessing randomly generated sequence
<400> 112
   Ser Pro Gly Met Cys Ala Arg
   1 5
<210> 113
   <211> 11
   <212> PRT
   <213> Artificial Sequence
<220>
   <221> MOD_RES
   <222> (8)
   <223> Stop codon but Glu when expressed in Tg-1 or JM109 strains of E. coli
<220>
   <223> Description of Artificial Sequence CDR1 and CDR3 inserts possessing randomly generated sequence
<400> 113
   Pro Phe Leu Phe Leu Pro Cys Glu Phe Phe Phe
   1 5 10
<210> 114
   <211> 11
   <212> PRT
   <213> Artificial Sequence
<220>
   <223> Description of Artificial Sequence CDR1 and CDR3 inserts possessing randomly generated sequence
<400> 114
   Trp Thr Leu Gly His His Lys Leu Cys Glu Gly
   1 5 10
<210> 115
   <211> 10
   <212> PRT
   <213> Artificial Sequence
<220>
   <223> Description of Artificial Sequence CDR1 and CDR3 inserts possessing randomly generated sequence
<400> 115
   Leu Phe Thr Cys Leu Leu Ala Leu Cys Ser
    1               5                  10
<210> 116
   <211> 7
   <212> PRT
   <213> Artificial Sequence
<220>
   <223> description of Artificial Sequence CDR1 and CDR3 inserts possessing randomly generated sequence
<400> 116
   Ser Pro Gly Glu Cys Tyr Gly
    1               5
<210> 117
   <211> 13
   <212> PRT
   <213> Artificial Sequence
<220>
   <223> Description of Artificial Sequence CDR1 and CDR3 inserts possessing randomly generated sequence
<400> 117
   Ser Trp Leu Ser Thr Thr Xaa Cys Leu Ser Ser Cys Ser
    1               5                  10
<210> 118
   <211> 7
   <212> PRT
   <213> Artificial Sequence
<220>
   <221> MOD_RES
   <222> (4)
   <223> Stop codon but Glu when expressed in Tg-1 or JM109 strains of E. coli
<220>
   <223> Description of Artificial Sequence CDR1 and CDR3 inserts possessing randomly generated sequence
<400> 118
   Ser Pro Gly Glu Cys Gln Asp
   1 5
<210> 119
   <211> 13
   <212> PRT
   <213> Artificial Sequence
<220>
   <223> Description of Artificial Sequence CDR1 and CDR3 inserts possessing randomly generated sequence
<400> 119
   Leu Leu Gly Ser Leu Leu Ser Cys Phe Ala Ser Leu Ser
   1 5 10
<210> 120
   <211> 13
   <212> PRT
   <213> Artificial Sequence
<220>
   <223> Description of Artificial Sequence CDR1 and CDR3 inserts possessing randomly generated sequence
<400> 120
   Ser Pro Gly Ser Leu Leu Ser Cys Phe Ala Ser Xaa Ser
   1 5 10
<210> 121
   <211> 7
   <212> PRT
   <213> Artificial Sequence
<220>
   <223> Description of Artificial Sequence CDR1 and CDR3 inserts possessing randomly generated sequence
<400> 121
   Ser Pro Gly Arg Cys Thr Asp
   1 5
<210> 122
   <211> 13
   <212> PRT
   <213> Artificial Sequence
<220>
   <223> Description of Artificial Sequence CDR1 and CDR3 inserts possessing randomly generated sequence
<400> 122
   Val Ile Cys His Ser Ser Val Cys Leu Ser Asp Val Cys
   1 5 10
<210> 123
   <211> 13
   <212> PRT
   <213> Artificial Sequence
<220>
   <223> Description of Artificial Sequence CDR1 and CDR3 inserts possessing randomly generated sequence
<400> 123
   Val Ile Cys His Ser Ser Val Cys Leu Ser Glu Val Cys
   1 5 10
<210> 124
   <211> 10
   <212> PRT
   <213> Artificial Sequence
<220>
   <223> Description of Artificial Sequence CDR1 and CDR3 inserts possessing randomly generated sequence
<400> 124
   Asp Leu Pro Ser Tyr Leu Ala Cys Ser Ile
   1 5 10
<210> 125
   <211> 7
   <212> PRT
   <213> Artificial Sequence
<220>
   <223> Description of Artificial Sequence CDR1 and CDR3 inserts possessing randomly generated sequence
<400> 125
   Ser Pro Gly Arg Cys Asp Ala
   1 5
<210> 126
   <211> 14
   <212> PRT
   <213> Artificial Sequence
<220>
   <223> Description of Artificial Sequence CDR1 and CDR3 inserts possessing randomly generated sequence
<400> 126
   Ala Leu Cys Trp Asp Val Phe Tyr Cys Ser Phe Pro Ser Tyr
   1 5 10
<210> 127
   <211> 11
   <212> PRT
   <213> Artificial Sequence
<220>
   <223> Description of Artificial Sequence CDR1 and CDR3 inserts possessing randomly generated sequence
<400> 127
   Glu Leu Phe Gly His Ala Arg Tyr Cys Lys Gly
   1 5 10
<210> 128
   <211> 15
   <212> PRT
   <213> Artificial Sequence
<220>
   <221> MOD RES
   <222> (7)
   <223> Stop codon but Glu when expressed in Tg-1 of JM019 strains of E. coli
<220>
   <223> Description of Artificial Sequence CDR1 and CDR3 inserts possessing randomly generated sequence
<400> 128
   Val Ser Ile Thr Ser Pro Glu Leu Cys Pro Val Lys Val Phe Asp
   1 5 10 15
<210> 129
   <211> 7
   <212> PRT
   <213> Artificial Sequence
<220>
   <221> MOD_RES
   <222> (6)
   <223> Stop codon but Glu when expressed in Tg-1 or JM109 strains of E. coli
<220>
   <223> Description of Artificial Sequence CDR1 and CDR3 inserts possessing randomly generated sequence
<400> 129
   Ser Pro Gly Lys Val Glu Asn
   1 5
<210> 130
   <211> 8
   <212> PRT
   <213> Artificial Sequence
<220>
   <223> Description of Artificial Sequence CDR1 and CDR3 inserts possessing randomly generated sequence
<400> 130
   Leu Phe Val Pro Phe Val Ser Pro
   1 5
<210> 131
   <211> 7
   <212> PRT
   <213> Artificial Sequence
<220>
   <223> Description of Artificial Sequence CDR1 and CDR3 inserts possessing randomly generated sequence
<400> 131
   Ser Pro Gly Asp Leu Trp Val
   1 5
<210> 132
   <211> 15
   <212> PRT
   <213> Artificial Sequence
<220>
   <223> Description of Artificial Sequence CDR1 and CDR3 inserts possessing randomly generated sequence
<400> 132
   Glu Ser Gly Leu Ser Pro Val Ser Pro Cys Ser Leu Tyr Ser Leu
   1 5 10 15
<210> 133
   <211> 8
   <212> PRT
   <213> Artificial Sequence
<220>
   <223> Description of Artificial Sequence CDR1 and CDR3 inserts possessing randomly generated sequence
<400> 133
   Thr Ser Ala Asn Gly Pro Tyr Gly
   1 5
<210> 134
   <211> 10
   <212> PRT
   <213> Artificial Sequence
<220>
   <223> Description of Artificial Sequence CDR1 and CDR3 inserts possessing randomly generated sequence
<400> 134
   Pro Trp Ala Tyr Arg Phe Leu Ala Val Leu
   1 5 10
<210> 135
   <211> 8
   <212> PRT
   <213> Artificial Sequence
<220>
   <223> Description of Artificial Sequence CDR1 and CDR3 inserts possessing randomly generated sequence
<400> 135
   Arg Lys Thr Arg Glu Lys Tyr Gly
   1 5
<210> 136
   <211> 12
   <212> PRT
   <213> Artificial Sequence
<220>
   <223> Description of Artificial Sequence CDR1 and CDR3 inserts possessing randomly generated sequence
<400> 136
   Glu Leu Met Tyr Pro Pro Pro Tyr Tyr Leu Gly Ile
   1 5 10
<210> 137
   <211> 7
   <212> PRT
   <213> Artificial Sequence
<220>
   <223> Description of Artificial Sequence CDR1 and CDR3 inserts possessing randomly generated sequence
<400> 137
   Ser Pro Gly Gln Glu Leu Thr
   1 5
<210> 138
   <211> 15
   <212> PRT
   <213> Artificial Sequence
<220>
   <223> Description of Artificial Sequence CDR1 and CDR3 inserts possessing randomly generated sequence .
<400> 138
   Glu Leu Phe Phe Leu Leu Tyr Ala Pro Cys Tyr Leu Phe Gln Arg
   1 5 10 15